# EUROPÄISCHE PATENTSCHRIFT

(11) **EP 1 228 777 B1**
(45) Veröffentlichungstag und Bekanntmachung des Hinweises auf die Patenterteilung: **29.10.2003**
(21) Anmeldenummer: 02008599.9
(22) Anmeldetag: 29.05.1995
(51) Int. Cl.: A61M 5/315

(54) **Injektionsgerät**
Injection device
Dispositif d'injection

(30) Priorität: 30.05.1994 DE 4418824
(43) Veröffentlichungstag der Anmeldung: 07.08.2002
(62) Teilanmeldung aus: 95921770.4
(73) Patentinhaber: B D Medico S.a.r.l., 1295 Mies (VD) (CH)
(72) Erfinder: Bechtold, Herbert, 71139 Ehningen (DE); Gabriel, Jochen, 70192 Stuttgart (DE)
(74) Vertreter: Raible, Hans, Dipl.-Ing.

(56) Entgegenhaltungen:
- EP-A- 0 295 075
- EP-A- 0 498 737
- WO-A-91/10460
- DE-A- 3 900 926
- US-A- 3 695 266

## Beschreibung

Die Erfindung betrifft ein Injektionsgerät, mittels dessen aus dem Inhalt eines im Injektionsgerät enthaltenen Vorratsbehälters mehrere Injektionen gegeben werden können. Solche Injektionsgeräte werden z.B. von Diabetikern verwendet, um sich regelmäßig Injektionen mit Insulin zu geben.

Bei Diabetes muss die zu injizierende Dosis, oft vor jeder Injektion, an den augenblicklichen Blutzuckerspiegel des Patienten angepasst werden. Aus der DE-C2, 3 645 245 kennt man zu diesem Zweck ein Injektionsgerät mit einstellbarer Dosis. Dieses hat eine Kolbenstange mit Außengewinde. Diese Kolbenstange ist im Gehäuse des Injektionsgeräts axial verschiebbar, kann aber relativ zum Gehäuse nicht verdreht werden. Ihr Außengewinde ist im Innengewinde eines drehbaren Teils geführt. Dreht man - zwecks Dosiseinstellung - dieses Teil, so bewirkt das eine axiate Verschiebung der Kolbenstange. Ein solches Injektionsgerät erlaubt eine exakte Dosierung der zu injizierenden Flüssigkeitsmenge, ist jedoch relativ teuer, weil für eine feine Dosierung ein entsprechend feines Gewinde erforderlich ist; auch ist die Bedienung kompliziert.

Aus der US-A, 4 659 327 kennt man ein Gerät zum Injizieren von Zahnzement aus einer vorgefertigten Kartusche. Dieses Gerät verwendet eine Zahn- bzw. Kolbenstange, die mit zwei gegenüber liegenden Zahnreihen versehen ist und die von einer Feder ständig in Auspressrichtung beaufschlagt wird, aber durch eine Mechanik an einer Bewegung in dieser Richtung gehindert wird. Mittels einer Taste kann der Zahnarzt diese Mechanik entsperren. Jeder Druck auf diese Taste bewirkt, dass sich die Zahnstange um genau eine Zahnteilung in Auspressrichtung bewegt und dadurch eine bestimmte Menge Zahnzement auspresst. Dies geschieht dadurch, dass die eine Zahnreihe der Zahnstange mit Sperrzähnen der Taste zusammenwirkt, und die andere Zahnreihe mit Sperrzähnen einer Klinke. Wollte man bei diesem Gerät die ausgepresste Menge Zahnzement verkleinern, so müsste man die Zahnteilung der beiden Zahnreihen verkleinern. Dieser Ansatz führte deshalb in Anwendungsgebieten, in denen eine sehr feine Dosierung gewünscht ist, zu einem hohen Produktionsauwand zur Herstellung entsprechend feiner Zahnreihen mit sehr geringen Abständen zwischen den Zähnen.

Es ist deshalb eine Aufgabe der Erfindung, ein neues Injektionsgerät bereitzustellen.

Nach der Erfindung wird diese Aufgabe gelöst durch den Gegenstand des Anspruchs 1. Werden z.B. zwei erste Eingriffsglieder verwendet, so ermöglicht die Erfindung alternativ einen vollen Eingriff des Eingriffselements des einen der beiden Eingriffsglieder mit der ihm zugeordneten Zahnreihe der Zahnstange, oder einen vollen Eingriff des Eingriffselements des anderen der beiden Eingriffsglieder mit der ihm zugeordneten Zahnreihe der Zahnstange, und dadurch eine verfeinerte Einstellmöglichkeit des Injektionsgeräts, ohne dass die Feinheit der Untergliederung einer der Zahnreihen erhöht werden müsste. Dies erweist sich besonders dann als Vorteil,wenn sehr kleine Injektionsdosen eingestellt werden sollen, welche Bewegungen des Kolbens in einem Vorratsbehälter entsprechen, die kleiner als 1 mm sind.

Injektionsgeräte für Diabetiker enthalten nämlich meist einen Vorratsbehälter in Form einer Standardkartusche mit 3 ml Insulin, wovon pro Injektion eine Menge injiziert wird, die zwischen 0,02 ml und 0,6 ml liegt. Dabei müssen sehr enge Toleranzen eingehalten werden, z.B. ±5 % bei einer Injektionsdosis von 0,2 ml (DIN/ISO 11608-1.3). Die Verwendung einer Mehrzahl von Zahnreihen und zugeordneter Eingriffsglieder ermöglicht es, bei Verwendung von Zähnen mit einer Zahnteilung, die bequem und genau gefertigt werden kann, eine Dosiseinstellung in Schritten vorzunehmen, die kleiner sind als diese Zahnteilung. Durch die Erfindung erreicht man also eine exakte Dosierung, verbunden mit der Möglichkeit, die Dosis in Schritten einzustellen, die kleiner sind als eine Zahnteilung. Erfindungsgemäße Injektionsgeräte können ggf. auch als preiswerte Wegwerfprodukte hergestellt werden.

Eine bevorzugte Weiterbildung der Erfindung ist Gegenstand des Anspruchs 5. Man erreicht so durch das Zusammenwirken der ersten und der zweiten Eingriffsglieder mit der Zahnstange, dass diese in sehr kleinen Schritten verschoben werden kann, wobei die zweiten Eingriffsglieder die Bewegung der Zahnstange in distaler Richtung begrenzen, und diese Begrenzung ebenfalls in Bruchteilen von Zahnteilungen erfolgen kann. Die zweiten Eingriffsglieder bilden dabei eine Art Rücklaufsperre für die Zahnstange, und sie werden bevorzugt ähnlich ausgelegt wie die ersten Eingriffsglieder, damit auch die Arretierung in Schritten erfolgen kann, die mit den Dosierschritten übereinstimmen, welche mit den ersten Eingriffsgliedern eingestellt werden können.

Eine bevorzugte Ausgestaltung dieser zweiten Eingriffsglieder ist dabei Gegenstand des Anspruchs 7.

Eine andere, außerordentlich vorteilhafte Weiterbildung der Erfindung ist Gegenstand des Anspruchs 12. Dies ermöglicht die notwendigen Justiervorgänge, denn die Vorratsbehälter (Kartuschen) für die Injektionsflüssigkeit sind zwar in ihrer Größe festgelegt, haben aber stets Fertigungstoleranzen, ebenso der in ihnen gewöhnlich angeordnete Kolben, und diese Toleranzen können durch die Veränderung der Länge des Halters in sehr einfacher Weise kompensiert werden.

Dazu weist der Halter gemäß Anspruch 13 mit besonderem Vorteil einen proximalen Abschnitt und einen distalen Abschnitt auf, welche durch eine verstellbare Verbindung miteinander verbunden sind.

Ferner wird der Halter mit Vorteil gemäß Anspruch 14 so ausgebildet, dass die verstellbare Verbindung mittels einer axialen Kraft verkürzbar ist, um die Gesamtlänge des Halters zu beeinflussen.

Gemäß Anspruch 15 wird die verstellbare Verbindung bevorzugt als Mikrorastung ausgebildet. Eine solche Mikrorastung, oder beispielsweise eine entsprechende Verschraubung mit einem Feingewinde, erlaubt eine Feineinstellung der Länge des Halters und damit eine sehr exakte Justierung, wie sie für eine exakte Einhaltung der Injektionsdosis wünschenswert ist.

In bevorzugter Weise ist dabei gemäß Anspruch 16 der hinsichtlich seiner Länge verstellbare Halter mit den zweiten Eingriffsgliedern fest verbunden.

Bei einer anderen bevorzugten Ausgestaltung der Erfindung gemäß Anspruch 18 ist zur Aufnahme des Vorratsbehälters für die zu injizierende Flüssigkeit ein Halter mit einem proximalen Abschnitt und einem distalen Abschnitt vorgesehen, welche Abschnitte durch eine Rastverbindung miteinander verbunden sind. Dies ermöglicht ein rasches Einsetzen des Vorratsbehälters in das Injektionsgerät, und eine rasche Montage.

Dabei verwendet man bevorzugt gemäß Anspruch 19 eine Rastverbindung, welche mehrere mögliche Raststellungen aufweist. Hierdurch ergibt sich eine sehr sichere und dabei leicht herstellbare Verbindung.

Ferner wird gemäß Anspruch 20 in bevorzugter Weise der distale Abschnitt des Halters mit den zweiten Eingriffsgliedern fest verbunden.

Weitere Einzelheiten und vorteilhafte Weiterbildungen der Erfindung ergeben sich aus den im folgenden beschriebenen und in der Zeichnung dargestellten, in keiner Weise als Einschränkung der Erfindung zu verstehenden Ausführungsbeispielen, sowie aus den übrigen Unteransprüchen. Es zeigen:
- Fig. 1 bis 5: Schaubilder, welche den zeitlichen Ablauf bei einem Injektionsvorgang in schematisierter Form wiedergeben,
- Fig. 6 und 7: eine zeichnerische Erläuterung von Einzelheiten der Fig. 1 bis 5,
- Fig. 8A und 8B: einen Längsschnitt durch eine bevorzugte Ausführungsform eines erfindungsgemäßen Injektionsgeräts, im nicht gespannten Zustand,
- Fig. 9A und 9B: Darstellungen analog Fig. 8, aber im gespannten Zustand,
- Fig. 10: eine schematische raumbildliche Darstellung des proximalen Endes des beim ersten und zweiten Ausführungsbeispiel verwendeten Stößels in Gestalt einer Zahnstange mit etwa quadratischem Querschnitt,
- Fig. 11A, B: einen Längsschnitt analog Fig. 8 und 9, aber in' einer zu diesen beiden Figuren senkrechten Ebene,
- Fig. 12: eine Darstellung, welche zeigt, wie ein Haken einer Verbindungsanordnung in den Stößel der Fig. 10 eingreift.
- Fig. 13: eine vergrößerte, schematisierte Darstellung der Rastung zwischen Teilen des Halters 54, wie sie auch beim nachfolgenden dritten Ausführungsbeispiel verwendet wird,
- Fig. 14: eine raumbildliche Darstellung eines Injektionsgeräts nach einem dritten Ausführungsbeispiel der Erfindung, bei welchem eine Verstellung der Injektionsdosis möglich ist,
- Fig. 15: eine raumbildliche Darstellung analog Fig. 14, wobei aber das Gehäuse zur besseren Darstellung teilweise im Schnitt dargestellt ist und im Bereich dieser Schnittdarstellung die inneren Teile des Injektionsgeräts nicht dargestellt sind,
- Fig. 16: eine raumbildliche, auseinandergezogene Darstellung von inneren Teilen des Injektionsgeräts der Fig. 14 und 15,
- Fig. 17: eine raumbildliche, auseinandergezogene Darstellung des Gehäuses der Fig. 14 und 15 und eines in diesem angeordneten Halterteils,
- Fig. 18: eine raumbildliche, auseinandergezogene Darstellung einer Kartusche, ihres Halters, und einer Injektionsnadel,
- Fig. 19 bis 21: verschiedene Darstellungen des Injektionsgeräts nach dem dritten Ausführungsbeispiel im Längsschnitt und bei verschiedenen Stellungen; die Spannfeder ist nicht dargestellt, und bei Fig. 21 ist der Auslöser (Clip), der in diesem Längsschnitt an sich nicht sichtbar wäre, um 45° versetzt gezeichnet,
- Fig. 22: einen Schnitt, gesehen längs der Linie XXII-XXII der Fig. 21,
- Fig. 23: einen Schnitt, gesehen längs der Linie XXIII-XXIII der Fig. 21, wobei im Schnitt nach Fig. 23 die inneren Teile des Injektionsgeräts nicht dargestellt sind,
- Fig. 24: eine Ausschnittsvergrößerung der Stelle XXIV der Fig. 19,
- Fig. 25: eine Ausschnittsvergrößerung der Stelle XXV der Fig. 20,
- Fig. 26: eine stark schematisierte raumbildliche Darstellung des beim dritten Ausführungsbeispiel verwendeten Stößels in Gestalt einer Zahnstange,
- Fig. 27: eine Darstellung, welche nur die obere und die untere Zahnreihe des Stößels der Fig. 26 zeigt,
- Fig. 28: eine Darstellung, welche nur die linke und die rechte Zahnreihe des Stößels der Fig. 26 zeigt,
- Fig. 29: eine schematische Darstellung zur Erläuterung des Vorschubvorgangs bei einem Vorschub um eine halbe Zahnteilung,
- Fig. 30: einen vergrößerten raumbildlichen Ausschnitt aus der in Fig. 31 dargestellten Zahnstange, wobei ein Teil der Zahnstange weggeschnitten dargestellt ist, um die Zahnung besser sichtbar zu machen, und
- Fig. 31: eine raumbildliche, vergrößerte Darstellung einer Zahnstange, wobei ein Teil weggeschnitten dargestellt ist, um die Zahnung besser sichtbar zu machen.

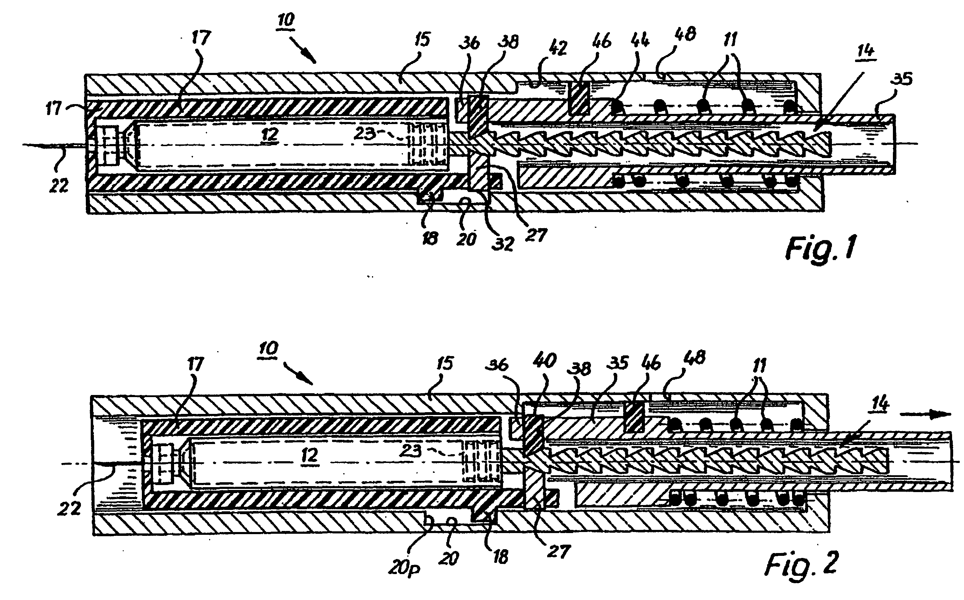

Die Fig. 1 bis 5 zeigen in stark schematisierter Form den Ablauf bei einem Injektionsvorgang.

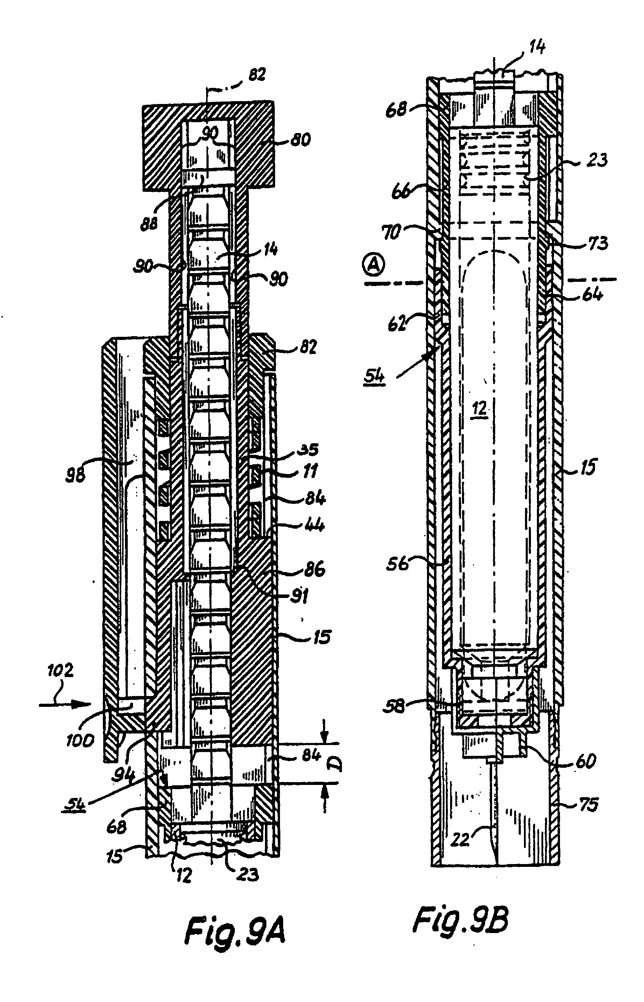

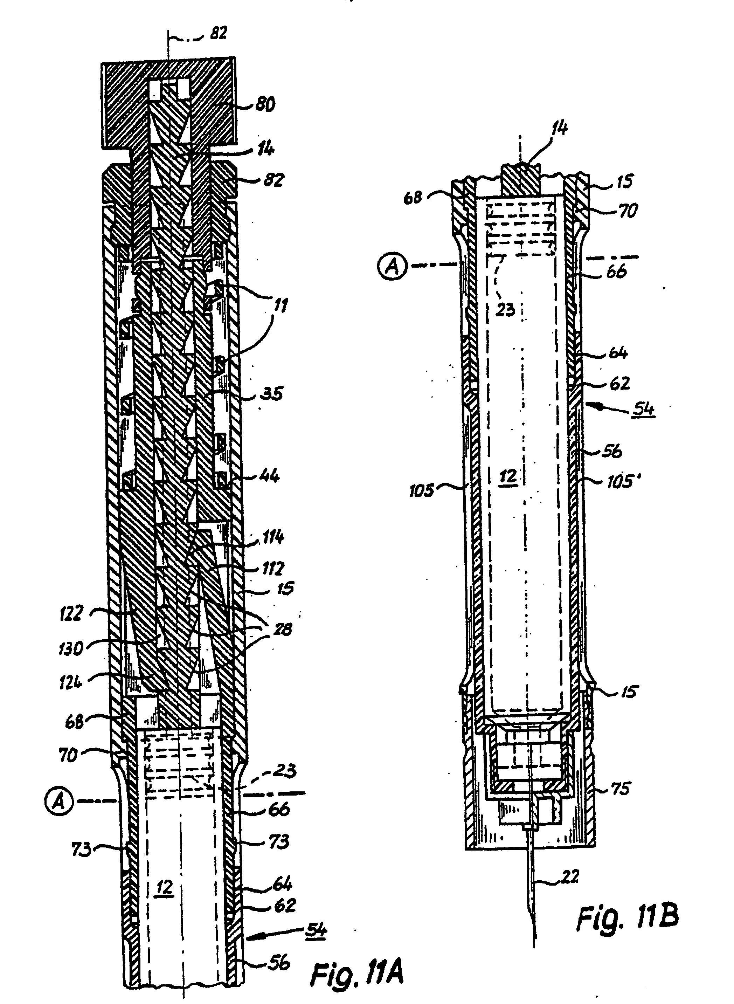

Fig. 1 zeigt das Injektionsgerät 10 in seiner Ruhelage bei nicht gespannter Feder 11 (analog der Darstellung nach den Fig. 8A und 8B, den Fig. 11A und 11B, sowie den Fig. 19 und 20). Fig. 2 zeigt den ersten Teil des Spannvorgangs der Feder 11, bei welchem ein Behälter (Kartusche) 12 für die zu injizierende Flüssigkeit aus seiner proximalen Endstellung (Fig. 1) in seine distale Endstellung (Fig. 2) gebracht wird, ohne daß hierbei ein Rastorgan 46 bereits in einer vorgegebenen Stellung (Spannstellung) in eine Rastöffnung 48 eingerastet wäre.

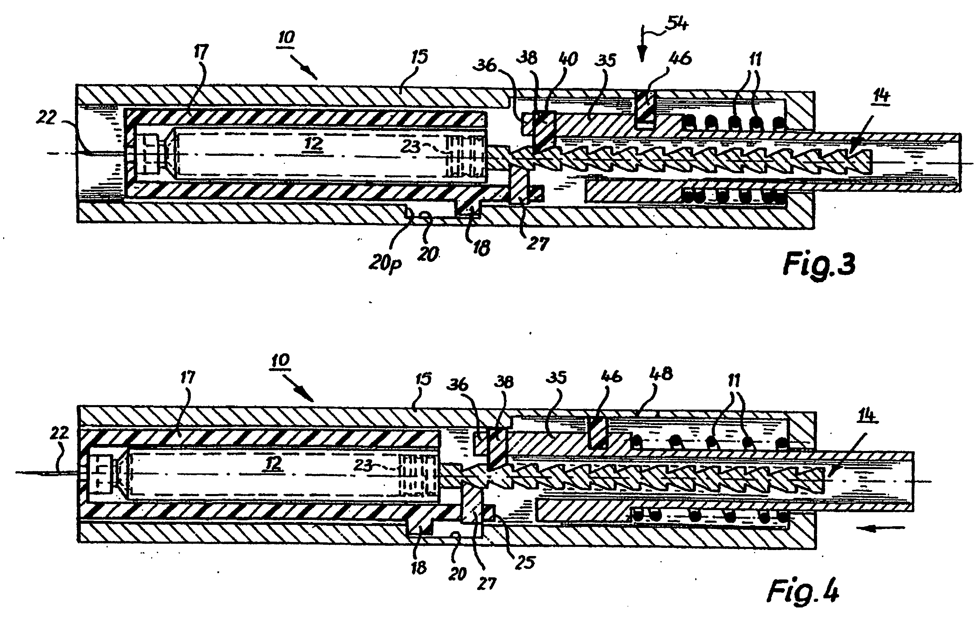

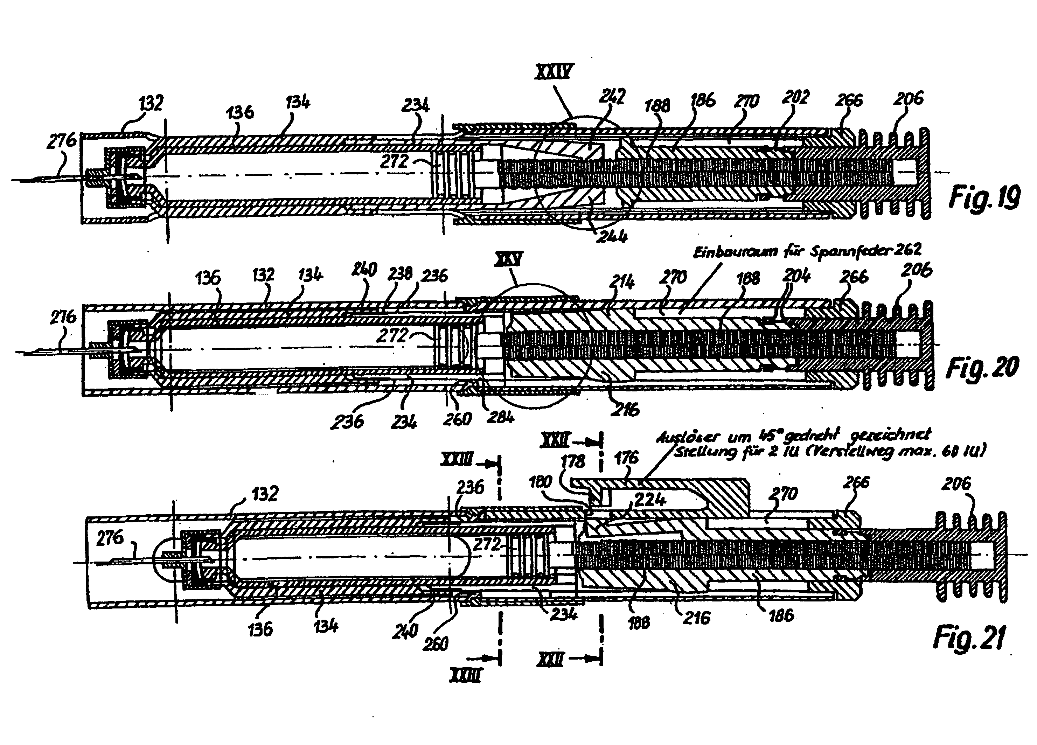

Fig.3 zeigt den Abschluß des Spannvorgangs, bei welchem die Feder 11 voll-gespannt und die Injektionsdosis eingestellt ist. Dies entspricht der Darstellung nach den Fig. 9A und 9B, bzw. der Darstellung nach Fig. 21.

Fig. 4 zeigt die erste Phase einer Injektion nach dem Auslösen eines Injektionsvorgangs, wobei die Nadel eingestochen, aber noch keine Flüssigkeit injiziert wird.

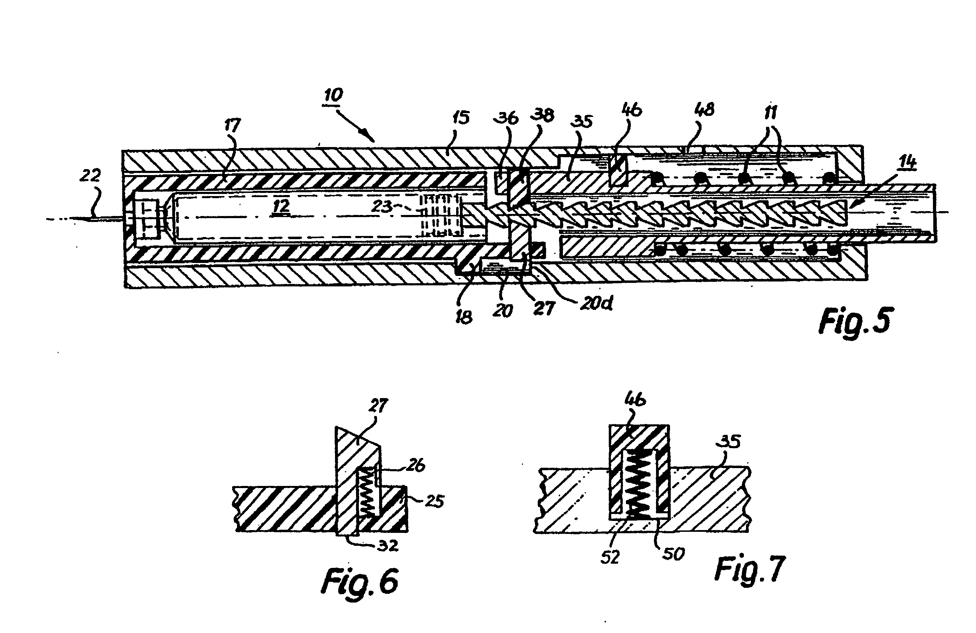

Fig. 5 zeigt die zweite Phase einer Injektion, nach dem Einstechen der Nadel, wobei in Fig. 5 der Zustand nach dem Injizieren der eingestellten Flüssigkeitsmenge dargestellt ist. Das Gerät befindet sich hier wieder in der Ruhelage gemäß Fig. 1, aber der (als Zahnstange ausgebildete) Stößel 14 ist gegenüber Fig. 1 um eine Zahnteilung in proximaler Richtung verschoben, entsprechend der injizierten Flüssigkeitsmenge.

Das Injektionsgerät 10 hat ein Gehäuse 15, in welchem der Behälter 12 in einem Halter 17 zwischen einer proximalen Endstellung (Fig. 1, 4 und 5) und einer distalen Endstellung (Fig. 2 und 3) axial verschiebbar angeordnet ist. Der Halter 17 hat hierzu einen radialen Vorsprung 18, der in einer entsprechenden Ausnehmung 20 des Gehäuses 15 in der dargestellten Weise zwischen zwei axialen Anschlagstellungen verschiebbar ist. Der Behälter 12 selbst ist im Halter 17 in geeigneter Weise fixiert. Der Behälter 12 ist gewöhnlich eine Ampulle aus Glas, an deren proximalem Ende eine Injektionsnadel 22 in der dargestellten, üblichen Weise befestigt werden kann, und in deren distalem Ende ein Kolben 23 verschiebbar angeordnet ist. Solche Ampullen (Kartuschen) werden in großen Stückzahlen produziert. Wird der Kolben 23 in proximaler Richtung verschoben, so wird Flüssigkeit aus dem Behälter 12 durch die Nadel 22 ausgepreßt.

Die Begriffe "proximal" und "distal" sind übliche medizinische Terminologie; sie bedeuten:
Proximal: Zum Patienten hin, also, bezogen auf Fig. 1 bis 5, in Richtung nach links.
Distal: Vom Patienten weg, also bezogen auf die Fig. 1 bis 5, in Richtung nach rechts.

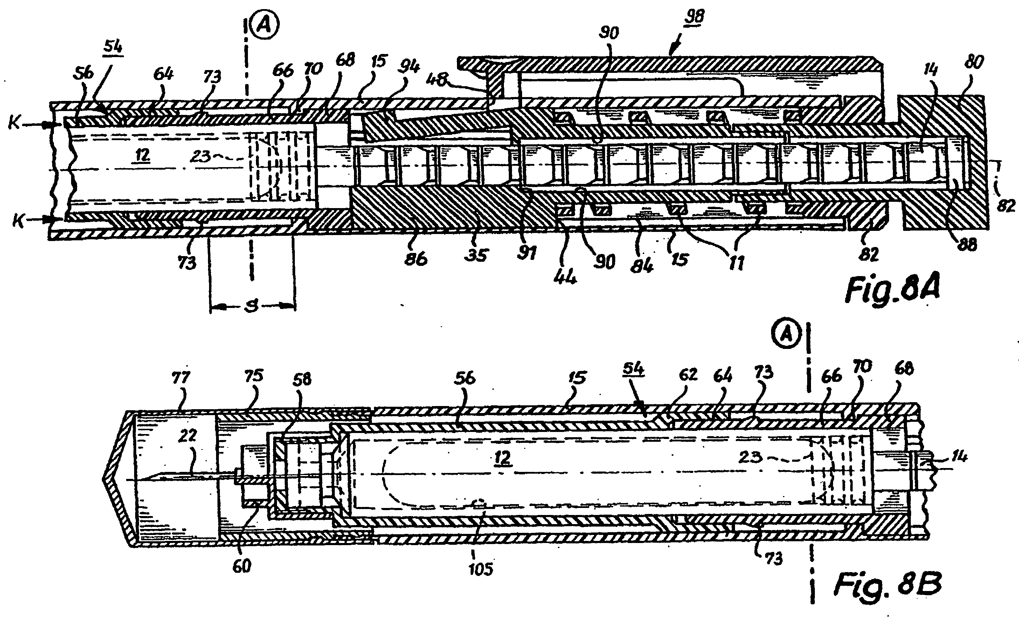

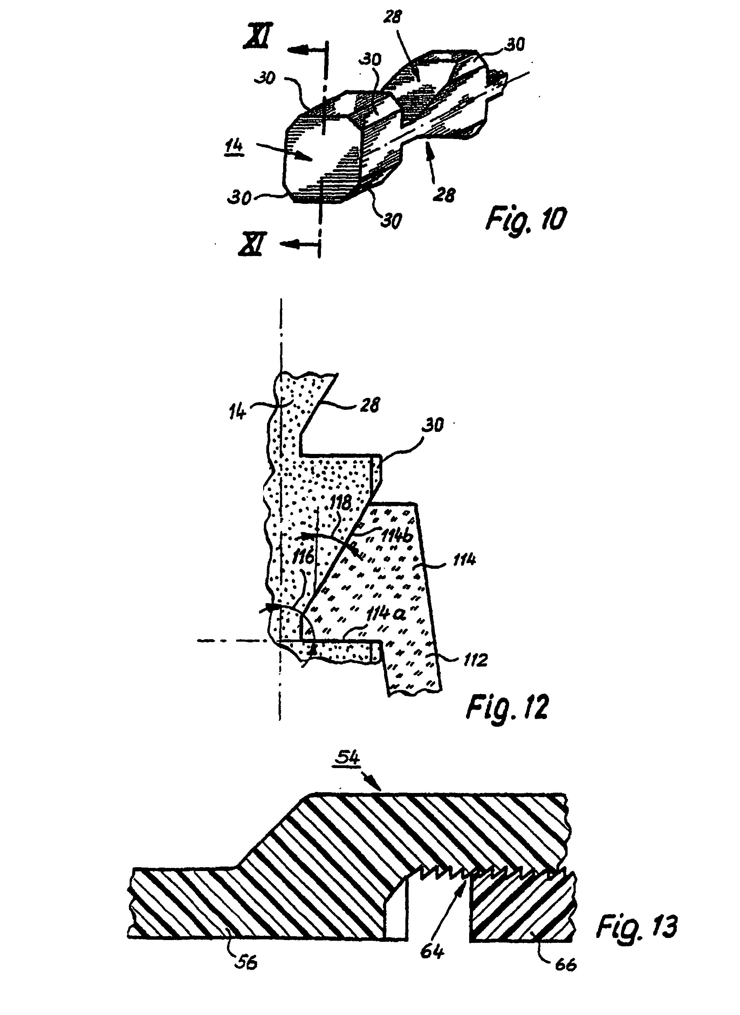

An einem in distaler Richtung sich erstreckenden Fortsatz 25 des Behälters 17 ist - als erste Verbindungsanordnung - ein Rastorgan in Form einer durch eine Feder 26 (Fig. 6) belasteten Klinke 27 angeordnet, und dieses Rastorgan ist so ausgebildet, daß es in eine Lücke der Zahnung 28 (Fig. 10) des Stößels 14 eingreifen kann, dessen bevorzugte raumbildliche Form aus Fig. 10 hervorgeht. Dieser Stößel 14 hat einen im wesentlichen quadratischen Querschnitt mit abgefasten Kanten 30, der auf zwei gegenüberliegenden Selten mit einer Zahnung 28 in der dargestellten Weise versehen ist, und die Klinke 27 paßt mit ihrer radial inneren Seite in die ihr zugewandte der Zahnung 28 des Stößels 14.

Die radial äußere Seite 32 (Fig. 6) der Klinke 27 wird gesteuert durch die Form der radialen Innenseite des Gehäuses 15. Bei der Stellung nach den Fig. 1, 4 und 5, also der proximalen Endstellung des Behälters 12, kann sich diese Klinke 27 radial nach außen bewegen, d.h., wenn der Stößel 14 hier in proximaler Richtung bewegt wird, wird die Klinke 27 radial nach außen verschoben und kann gemäß Fig. 5 in die nächste Zahnlücke 28 gleiten.

Dagegen ist außerhalb der proximalen Endstellung des Behälters 12 die Klinke 27 durch die Innenseite des Gehäuses 15 an einer Bewegung nach außen gehindert, wie das die Fig. 2 und 3 klar zeigen, und so entsteht in diesem Stellungsbereich eine direkte Antriebsverbindung zwischen dem Stößel 14 und dem Halter 17, welche eine Bewegung des Stößels 14 in proximaler Richtung direkt auf den Behälter 12 und die Nadel 22 Überträgt. Diese Antriebsverbindung wird erst unterbrochen, wenn der Behälter 12 seine proximale Endstellung (Fig. 1, 4 und 5) erreicht hat, da erst dann die Klinke 27 in der bereits ausführlich beschriebenen Welse radial nach außen verschoben werden kann. Man kann dies auch als Kulissensteuerung oder Nockensteuerung der Klinke 27 bezeichnen.

Hier ist auch darauf hinzuweisen, daß als Rastorgan zum Eingriff in die Lücken der Zahnung 28 viele Varianten möglich sind. Es handelt sich jeweils um federnd auslenkbare Teile, deren federnde Auslenkung außerhalb der proximalen Endstellung gesperrt ist, also um eine wegabhängige Steuerung dieser federnden Auslenkbarkeit, die selbstverständlich auf sehr viele verschiedene Arten möglich ist.

Der Stößel, 14 ist umgeben von einem Antriebsorgan 35, das hier beispielhaft etwa nach Art eine Rohres ausgebildet ist, das um den Stößel 14 herum angeordnet ist und zu dessen Antrieb dient, und zwar über eine zweite Verbindungsanordnung.

Hierzu ist in einem axialen Fortsatz 36 des Antriebsorgans 35 ein Rastorgan in Gestalt einer radial verschiebbaren, federbelasteten Klinke 38 vorgesehen, die genauso aufgebaut ist wie die in Fig. 6 dargestellte Klinke 27, also gegen die Kraft einer Feder radial nach außen verschoben werden kann. Die Klinke 38 greift in einen der Zähne 28 des Stößels 14 ein, und zwar, wie dargestellt, auf der in Fig. 1 oberen Seite, während die Klinke 27 auf der in Fig. 1 unteren Seite in eine der lücken der Zahnung 28 des Stößels 14 eingreift. (Dies ist der Grund, warum der Stößel 14 auf verschiedenen Seiten mit Zähnen bzw. Zahnlücken versehen ist, bevorzugt auf zwei gegenüberliegenden Seiten.)

Das radial äußere Ende 40 (Fig. 2) der Klinke 38 wird gesteuert von der ihr gegenüberliegenden Innenseite 42 (Fig. 1) des Gehäuses 15. Wie ein Vergleich der Fig. 1 und 2 zeigt, ist eine radiale Bewegung der Klinke 38 im proximalen Endstellungsbereich blockiert, d.h. in diesem Bereich wird eine Bewegung des Antriebsorgans 35 in beiden Richtungen voll auf den Stößel 14 übertragen.

Bei Erreichen der Stellung nach Fig. 2 befindet sich der Halter 17 mit dem Behälter 12 in seiner distalen Endstellung (durch Anschlag des Vorsprungs 18 am distalen Ende 20d der Gehäuseausnehmung 20). Ab dieser Stelle wird die radiale Bewegung der Klinke 38 nicht mehr gesperrt, da ab hier die Innenseite 42 des Gehäuses 15 einen größeren Durchmesser hat. Ab hier kann also die Klinke 38 über einen Zahn des Stößels 14 hinweg in die nächste Zahnlücke gleiten, wie das Fig. 3 zeigt, und dort einrasten.

Da die Spannfeder 11 zwischen dem distalen Ende des Gehäuses 15 und einer Schulter 44 (Fig. 1) des Antriebsorgans 35 angeordnet ist, wird sie bei einer distalen Verschiebung des Antriebsorgans 35 gespannt, und bei Erreichen der Spannstellung greift ein am Antriebsorgan 35 angeordnetes Rastglied 46 in die Ausnehmung 48 des Gehäuses 15 ein und verrastet dort das Antriebsorgan 35 in der Stellung gemäß Fig. 3. Dies ist die Stellung vor einer Injektion. Hierbei befindet sich die Nadel 22 im Gehäuse 15, ist also für den Benutzer nicht sichtbar, was ihm die Angst vor einer Injektion nimmt. Der Halter 17 ist in dieser Stellung im Gehäuse 15 festgehalten, weil er durch die Klinke 27 mit dem Stößel 14 fest verbunden ist, und weil der Stößel 14 seinerseits durch die Klinke 38, die ja mit elastischer Vorspannung gegen den Stößel 14 anliegt, gegen ungewünschte axiale Verschiebungen relativ zum Antriebsorgan 35 gesichert ist, das seinerseits durch das Rastglied 46 im Gehäuse 15 blockiert ist.

Fig. 7 zeigt einen möglichen Aufbau des Rastglieds 46, das hier in einer Ausnehmung 50 des Antriebsorgans 35 radial verschiebbar angeordnet und durch eine Druckfeder 52 radial nach außen beaufschlagt ist. Die Fig. 8 und 9 zeigen eine andere, bevorzugte Ausgestaltung dieses Rastglieds, und diese wird nachfolgend beschrieben werden.

Wird in der Stellung nach Fig. 3 durch Druck auf das Rastglied 46 in Richtung des Pfeiles 54 eine Injektion ausgelöst, so verschiebt die Feder 11 das Antriebsorgan 35 in proximaler Richtung. Über die Klinke 40 wird diese Bewegung direkt auf den Stößel 14 übertragen, und dieser überträgt seinerseits über die Klinke 27, die nicht radial nach außen ausweichen kann, diese Bewegung direkt auf den Halter 17 und den Behälter 12, so daß der Behälter 12 in proximaler Richtung verschoben und die Nadel 22 eingestochen wird, wie das Fig. 4 zeigt.

Fig. 4 zeigt auch, daß dann, wenn der Behälter 12 seine proximale Endstellung erreicht, wobei sein Vorsprung 18 zum Anschlag gegen das proximale Ende 20p der Ausnehmung 20 kommt, die Klinke 27 in den Bereich der Ausnehmung 20 auf der Innenseite des Gehäuses 15 gelangt und folglich jetzt radial nach außen ausweichen kann, d.h. der Stößel 14 kann seine proximale Bewegung fortsetzen und verschiebt jetzt den Kolben 23 im Behälter 12 in proximaler Richtung, und zwar um einen Zahnabstand des Stößels 14, wodurch eine entsprechende Flüssigkeitsmenge aus dem Behälter 12 ausgepreßt und injiziert wird. Der Stößel 14 kann z.B. 10 oder 14 Zähne haben, je näch der Größe der gewünschten Injektionsdosis.

Ist nach mehreren Injektionen die gesamte Flüssigkeit aus dem Behälter 12 ausgepreßt, so ist das Injektionsgerät 10 ausgebraucht und kann anschließend stofflich wiederverwertet werden. Mit einem Stößel 14 mit-wie dargestellt - 14 Zähnen lassen sich also 14 Injektionen mit identischer Dosis verabreichen, und der Vorgang der Injektion läuft nach dem Auslösen automatisch und mit sehr guter Dosiergenauigkeit ab, da die eigentliche Injektion erst beginnt, nachdem die Nadel 22 eingestochen wurde.

Die Fig. 8 bis 13 zeigen eine bevorzugte Ausführungsform der Erfindung. Gleiche oder gleichwirkende Teile wie in den vorhergehenden Figuren werden gewöhnlich mit denselben Bezugszeichen bezeichnet und dann nicht oder nur noch kurz beschrieben.

Wie die Fig. 8B und 13 zeigen, ist der Halter 54 für den Behälter 12 hier zweiteilig ausgebildet. Er hat einen proximalen Abschnitt 56, an dessen proximalem Ende ein Gewinde 5B zum Aufschrauben eines an der Nadel 22 befestigten Kunststoffteils 60 vorgesehen ist, wie das Fig. 8B klar zeigt. Am distalen Ende hat dieser proximale Abschnitt 56 eine Erweiterung 62, die innen mit einer Mikrorastung 64 versehen ist, und in diese greift ein distaler Abschnitt 66, dessen proximales Ende außen ebenfalls mit einer Mikrorastung versehen ist, welche in die Mikrorastung 64 eingreift. Die Mikrorastung 64 ist in Fig. 13 nur schematisch dargestellt, denn bevorzugt hat sie eine sehr feine Zahnteilung von z.B. 0,1 mm, die zeichnerisch nicht dargestellt werden kann. Die Abschnitte 56 und 66 nehmen in sich den Behälter 12 (mit seinem Kolben 23) in der dargestellten Weise auf. Dies ist ein Standardbehälter, wie er von verschiedenen Firmen hergestellt wird.

Der Aufbau des Halters 54 aus zwei Abschnitten 56 und 66 hat folgenden Grund: Die Behälter 12 und ihre Füllmenge unterliegen den üblichen Schwankungen, so daß bei der Fertigung die Lage des Kolbens 23 relativ zum Halter 54 immer innerhalb gewisser Toleranzgrenzen schwankt.

Es ist aber wichtig, daß bereits vor der ersten Injektion das proximale Ende des Stößels 14 direkt gegen den Kolben 23 anliegt, so, wie in Fig. 8 dargestellt, denn wenn zwischen dem Kolben 23 und dem proximalen Ende des Stößels 14 vor der ersten Injektion ein Spalt vorhanden ist, reduziert sich die erste Injektionsmenge entsprechend, und der Patient erhält zu wenig von der Flüssigkeit, die er injizieren sollte. Deshalb muß ein solcher Spalt vermieden werden.

Dies geschieht dadurch, daß mittels der Mikrorastung 64 der proximale Abschnitt 56 des Halters 54 so lange relativ zum distalen Abschnitt 66 verschoben wird, bis der Kolben 23 ohne Spalt gegen das proximale Ende des Stößels 14 anliegt. Durch die Mikrorastung 64 bleibt anschließend diese Verbindung der Abschnitte 56 und 66 erhalten.

Der distale Abschnitt 66 hat einen Abschnitt 68 größeren Durchmessers, mit dem er in der proximalen Endstellung gegen einen Bund 70 auf der Innenseite des Gehäuses 15 anliegt. Mit diesem Abschnitt 68 ist er auch In einer Längsnut 84 des Gehäuses 15 geführt und gegen Verdrehung gesichert.

Ferner hat der distale Abschnitt 66 auf seiner Außenseite einen Anschlag 73, mit dem er in der distalen Endstellung, welche in Fig. 9B dargestellt ist, gegen den Bund 70 anliegt. Die axiale Bewegung des Halters 54 erfolgt, wie bei den Fig. 1 bis 5 beschrieben, durch die stellungsabhängige Antriebsverbindung mit dem Stößel 14, die nachfolgend anhand der Fig. 11A und 11B beschrieben wird. Der Gesamthub S des Halters 54 ist in Fig. 8A zur Verdeutlichung angegeben.

Das Gehäuse 15 ist bei Fig. 8B mit einer Hülse 75 für die Stichtiefeneinstellung versehen, und auf diese Hülse kann in der dargestellten Weise eine Schutzkappe 77 aufgesetzt werden, um die Nadel 22 zu schützen. Wie Fig. 9B zeigt, befindet sich in der gespannten Stellung die Nadel 22 innerhalb der Hülse 75.

Es ist ferner darauf hinzuweisen, daß in der Ruhelage das Antriebsorgan 35 mit seinem proximalen Ende, und beaufschlagt durch die Spannfeder 11, gegen das distale Ende des Halters 54 anliegt, wie das in Fig. 8A dargestellt ist. (In Fig. 8B ist dies nicht dargestellt.) Dies ermöglicht es, bei der erwähnten Einstellung der Mikrorastung 64 mit einer Kraft K (links in Fig. 8A) in distaler Richtung auf den proximalen Abschnitt 56 des Halters 54 zu drücken und dadurch den Spalt zwischen dem Kolben 23 und dem proximalen Ende des Stößels 14 auf Null zu reduzieren.

Man erkennt auch in Fig. 9A, daß dort ein Abstand D zwischen dem distalen Ende des Halters 54 und dem proximalen Ende des Antriebsorgans 35 liegt, und dieser Abstand D entspricht der zu injizierenden Flüssigkeitsmenge, also hier dem Abstand zwischen zwei Zähnen 28.

Das Antriebsorgan 35 ist bei der Ausführungsform nach den Fig. 8 bis 13 mit einem Ziehknopf 80 zum Spannen der Feder 11 versehen, welcher in der dargestellten Weise mit dem Antriebsorgan 35 fest verbunden ist und mit diesem eine Einheit bildet. (Dieser Knopf ist in den Fig. 1 bis 5 nicht dargestellt.)

Die Längsachse des Injektionsgeräts 10 ist in den Fig. 8 und 9 mit 82 bezeichnet. Es ist darauf hinzuweisen, daß die Darstellungen der Fig. 8 und 9 sehr stark vergrößert sind, d.h. das gesamte Gerät hat etwa die Größe eines überdimensionierten Füllfederhalters mit einer Länge von z.B. 16 bis 17 cm.

Die Spannfeder 11 ist bei der Ausführungsform nach den Fig. 8 bis 13 als Spritzgußteil (Schraubenfeder) aus Kunststoff ausgebildet, und sie ist bevorzugt einstückig mit einem Teil 82, welches in der dargestellten Weise den distalen Abschluß des Gehäuses 15 bildet und mit diesem in der dargestellten Weise verbunden ist. Die Feder 11 liegt mit ihrem proximalen Ende gegen die Schulter 44 des Antriebsorgans 35 an. Letzteres ist in einer entsprechenden zylindrischen Ausnehmung des Teils 82 geführt, wie in Fig. 8A und 9A dargestellt. Es ist ferner mit einer radialen Verbreiterung 86 in der Längsnut 84 des Gehäuses 15 geführt, so daß es sich im Gehäuse 15 nicht drehen kann.

Ebenso ist der Stößel 14 mit einer Verbreiterung 88 an seinem distalen Ende in zwei Längsnuten 90 im Inneren des Antriebsgliedes 35 bzw. des mit ihm verbundenen Knopfes 80 geführt. Diese Längsnuten 90 erstrecken sich bis zu einer Stelle 91. Gelangt die Verbreiterung 88 zu dieser Stelle 91, so kann der Stößel 14 nicht mehr weiter aus dem Antriebsglied 35 herausgeschoben werden, und dadurch wird der bei Fig. 3 dargestellte Betätigungsschritt gesperrt, d.h. das Gerät kann nicht mehr in die Raststellung gemäß Fig. 3 gebracht werden. Dadurch erkennt der Benutzer, daß keine weitere Injektion mehr möglich ist.

Zum Zwecke der Verrastung in der Spannstellung ist am Antriebsorgan 35 ein elastisch nach innen auslenkbarer Rasthaken 94 vorgesehen, der in der dargestellten Weise einstückig mit dem Antriebsorgan 35 ausgebildet ist und wie dieses aus einem elastischen Kunststoff ausgebilde ist. In der Stellung nach Fig. 8A liegt er federnd gegen die Innenseite des Gehäuses 15 an, und in der Raststellung gemäß Fig. 9A, in der die Feder 11 gespannt ist, ist er in die Rastausnehmung 48 (Fig. 8A) im Gehäuse 15 eingerastet.

Zum Auslösen des Injektionsgeräts dient ein Clip 98, welcher, der Rastausnehmung 48 gegenüberliegend, mit einem radial nach innen ragenden Vorsprung 100 versehen ist. Wird der Clip 98, der aus elastischem Kunststoff ausgebildet ist, in Richtung des Pfeiles 102 (Fig. 9A) nach innen gedrückt, so drückt er den Rasthaken 94 aus der Rastausnehmung 48 (Fig. 8A) heraus und löst so einen Injektionsvorgang aus, dessen Ablauf anhand der Fig. 4 und 5 bereits ausführlich beschrieben wurde. Auch der Spannvorgang wurde bereits anhand der Fig. 1 bis 3 ausführlich beschrieben.

Der Clip 98 ist bevorzugt einstückig mit dem Teil 82 und der Feder 11 ausgebildet, was die Herstellung sehr vereinfacht. Das Antriebsorgan 35, der Knopf 80, der Stößel 14 und das Gehäuse 15 sind bevorzugt aus demselben Kunststoff hergestellt wie das Teil 82, die Feder 11 und der Clip 98, was die stoffliche Wiederverwertung des Injektionsgeräts außerordentlich vereinfacht, da der ganze distale Teil des Geräts en bloc der wiederverwertung zugeführt werden kann. Lediglich für den Halter 54 wird ein transparenter Kunststoff benötigt, damit der Inhalt des Behälters 12 von außen, sichtbar ist (ein Fenster 105 im Gehäuse 15 ist in Fig. 8B angedeutet). Deshalb muß der Halter 54 getrennt entsorgt werden, ebenso naturgemäß der Behälter 12, der gewöhnlich aus Glas hergestellt wird.

In Fig. 8, 9 und 11 ist zur Erleichterung der Orientierung eine bestimmte Stelle des Injektionsgeräts mit A bezeichnet. Wie man sieht, überlappen sich die Darstellungen jeweils im Mittelbereich.

Die Fig. 11A und 11B zeigen einen Schnitt, gesehen in Richtung der Linie XI-XI der Fig. 10. Das Injektionsgerät befindet sich bei dieser Darstellung in einer Lage gemäß Fig. 8A und 8B, also In der Lage nach einer Injektion, d.h. die Nadel 22 ragt aus der Hülse 75 heraus. Wie Fig. 11B zeigt, ist das Gehäuse 15 im Bereich der Kartusche 12 mit zwei gegenüberliegenden Fenstern 105, 105' versehen, und da die Teile des Halters 54 aus einen transparenten Kunststoff hergestellt sind, ist es möglich, durch diese Fenster zu sehen, wieviel Flüssigkeit noch im Behälter 12 enthalten ist.

Mit dem distalen Abschnitt 66 des Halters 54 ist gemäß Fig. 11A ein federnder Fortsatz 112 verbunden, der In distaler Richtung und unter einem Winkel von z.B. 10° schräg nach innen verläuft und der an seinem freien, distalen Ende einen Haken 114 hat und mit diesem in eine Zahnlücke 28 des Stößels 14 eingreift. Fortsatz 112 und Haken 114 dienen im Zusammenwirken mit den Zahnlücken 28 des Stößels 14 als eine erste Verbindungsanordnung, welche zwischen dem Stößel 14 und dem Halter 54 wirksam ist. Wie man aus Fig. 12 ersieht, verläuft die proximale Seite 114a des Hakens 114 unter einem Winkel 116 zum Stößel 14, und die distale Schrägseite 114b des Hakens 114 verläuft unter einem Winkel 118 zum Stößel 14. Der Winkel 116 liegt in der Größenordnung von 90°, und der Winkel 118 bevorzugt in der Größenordnung von 15 bis 30°. Ein Winkel von 23° hat sich bei Versuchen als günstig erwiesen.

Bewegt sich der Stößel 14 in distaler Richtung, so zieht er den Haken 114, und mit ihm den Halter 54, In distaler Richtung mit, wodurch sich die Stellung des Halters 54 gemäß Fig. 9B ergibt. Hierbei liegt also eine formschlüssige Verbindung zwischen dem Stößel 14 und dem Haken 114 vor.

Bewegt sich - bei einem Injektionsvorgang - der Stößel 14 in proximaler Richtung, so wird der Haken 114, der ja mit seiner Seite 114b mit Vorspannung gegen den Stößel 14 anliegt, ebenfalls in proximaler Richtung verschoben, d.h. es entsteht eine teils form-, teils kraftschlüssige Verbindung zwischen dem Stößel 14 und dem Haken 114, und der Halter 54 wird solange in der proximalen Richtung bewegt, bis die Stellung gemäß Fig. 11B erreicht ist, in welcher der Abschnitt 68 des Halters 54 gegen den Bund 70 anliegt und eine weitere proximale Bewegung des Halters 54 verhindert. Bewegt sich nun der Stößel 14 in proximaler Richtung weiter, so gleitet der Haken 114 aus der Zahnlücke 28 heraus und in die darauffolgende Zahnlücke 28 des Stößels 14, d.h. die erste Verbindungsanordnung wird unterbrochen, nachdem die Nadel 22 eingestochen wurde, damit nun der Stößel 14 den Kolben 23 in proximaler Richtung verschieben kann. Hierdurch wird die eingestellte Flüssigkeitsmenge aus dem Behälter 12 ausgepreßt und in den Patienten injiziert.

Vom Antriebsorgan 35 ragt ein federnder Fortsatz 122 in proximaler Richtung schräg nach innen, und dieser ist an seinem freien, proximalen Ende mit einer an die Zahnung 28 angepaßten Spitze 124 versehen, die - wie dargestellt - in eine Zahnlücke 28 auf der in Fig. 11A linken Seite des Stößels 14 eingreift. Im Zusammenwirken mit den Zahnlücken 28 wirken der Fortsatz 122 und die Spitze 124 als eine zweite Verbindungsanordnung, die zwischen dem Antriebsorgan 35 und dem Stößel 14 wirksam ist.

Auch hier gilt die schematische Darstellung der Fig. 12 hinsichtlich der Winkel und der bevorzugten Werte dieser Winkel, sofern die Zähne bzw. Zahnlücken 28 auf beiden Seiten des Stößels 14 gleich ausgebildet sind. (Diese können bei Bedarf unterschiedliche Formen und unterschiedliche Winkel haben). Günstig ist es, wenn die Teile 114, 124 von entgegengesetzten Seiten federnd, d.h. mit Vorspannung, gegen den Stößel 14 anliegen, da dieser dann nicht nach einer Seite gebogen wird.

Wird der Ziehknopf 80 in distaler Richtung verschoben, so folgt die Spitze 124 dieser Bewegung, und da sie kraftschlüssig mit dem Stößel 14 verbunden ist, verschiebt sie den Stößel 14 in distaler Richtung. Der Stößel 14 zieht seinerseits Über den Haken 114 den Halter 54 in distaler Richtung bis zum Anschlag des Anschlagteils 73 gegen den Bund 70. Dabei durchläuft der Halter 54 den in Fig. 8A dargestellten Weg S.

Beim Anschlag des Teils 73 gegen den Bund 70 wird eine weitere distale Bewegung des Halters 54 blockiert, ebenso eine weitere distale Verschiebung des Stößels 14 (durch den Eingriff des Hakens 114 In eine Zahnlücke 28). Wird nun bei der Spannbewegung am Ziehknopf 80 weiter gezogen, so wird die kraftschlüssige Verbindung zwischen der Spitze 124 und der Zahnlücke 28 gelöst, und die Spitze 124 gleitet in die darauffolgende Zahnlücke 130 des Stößels 14. Auf diese Weise wird die nächste Injektionsdosis eingestellt, die dem Abstand zwischen zwei aufeinanderfolgenden Zähnen bzw. Zahnlücken 28 entspricht. Dabei rastet, wie bereits beschrieben, der Rasthaken 94 In die Rastausnehmung 48 ein.

Beim Auslösen des Injektionsgeräts durch Druck auf den Clip 98 (Fig. 9A) erzeugt die Feder 11 eine Kraft in proximaler Richtung auf die Spitze 124, die sich jetzt in der Zahnlücke 130 befindet. Die Spitze 124 verschiebt durch die formschlüssige Verbindung den Stößel 14 in proximaler Richtung, und diese Bewegung wird, wie bereits beschrieben, durch die kraftschlüssige Verbindung zwischen Stößel 14 und Haken 114 auf den Halter 54 übertragen, wodurch die Nadel 22 eingestochen wird. Nach dem Einstechen wird die kraftschlüssige Verbindung zwischen dem Stößel 14 und dem Haken 114 unterbrochen, und der Stößel 14 verschiebt den Kolben 23 um die eingestellte Distanz, also die Distanz zwischen zwei aufeinanderfolgenden Zähnen bzw. Zahnlücken 28, und bewirkt dadurch eine Injektion der eingestellten Flüssigkeitsmenge.

Es ist darauf hinzuweisen, daß der Haken 114 und die Spitze 124 auch durch entsprechende (nicht dargestellte). Teile im Inneren des Gehäuses 15 in bestimmten axialen Stellungen an einer Bewegung radial nach außen gehindert werden können, wie das anhand der Fig. 1 bis 6 in großer Ausführlichkeit erläutert wurde und für den Fachmann sofort verständlich ist. Jedoch genügt in vielen Fällen auch die dargestellte und beschriebene Ausführungsform gemäß Fig. 11A für einen zuverlässigen Betrieb, sofern die Vorspannkräfte der Fortsätze 112, 122 und die Winkel der Zähne 28 richtig gewählt sind. Versuche haben gezeigt, daß die Ausführungsform nach Fig. 11A durchaus zufriedenstellend und zuverlässig arbeitet. In vielen Fällen wird man jedoch aus Sicherheitsgründen im Gehäuse Kulissen vorsehen, welche analog zum Ausführungsbeispiel nach den Fig. 1 bis 6 die radialen Bewegungen der Fortsätze 112 und 122 in bestimmten Stellungen limitieren und dadurch Fehlfunktionen mit absoluter Sicherheit vermeiden.

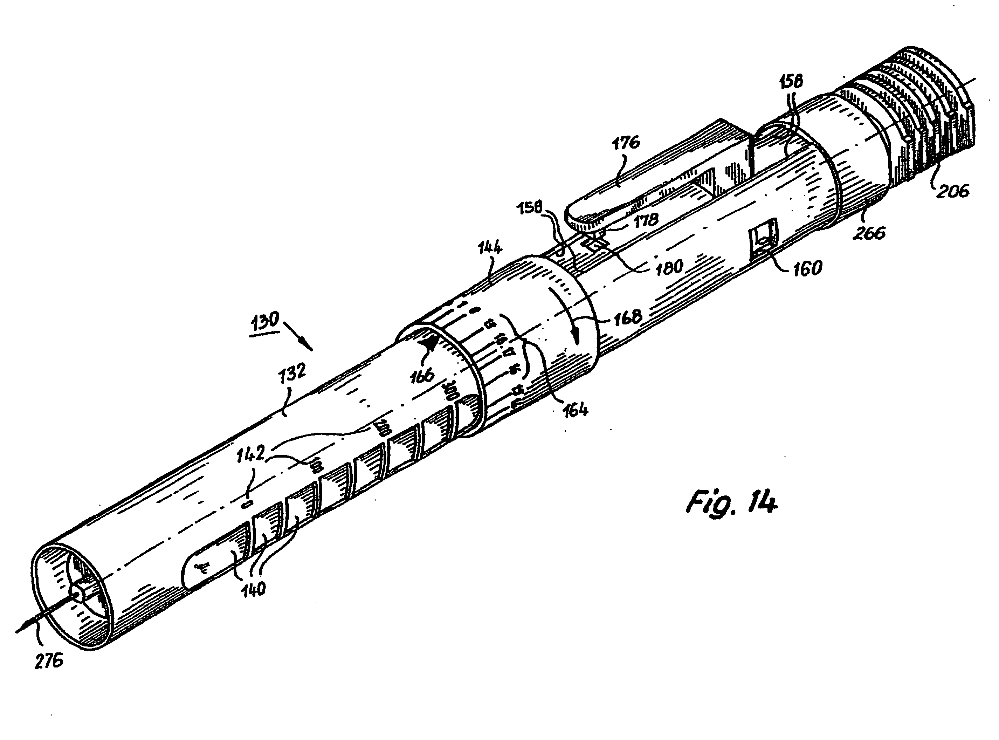

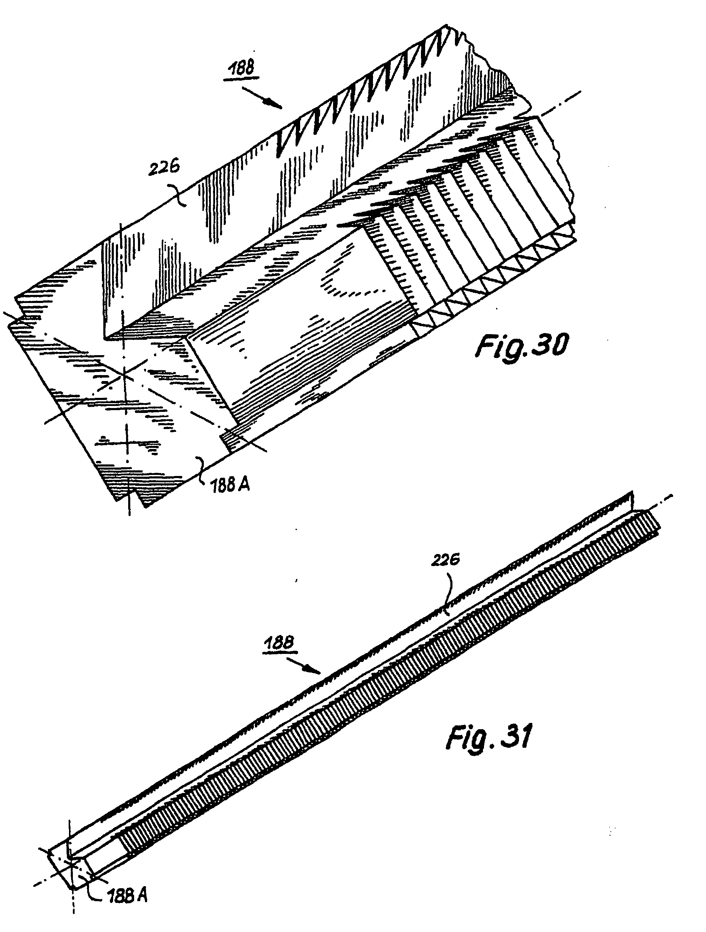

Die Fig. 14 bis 31 zeigen ein drittes Ausführungsbeispiel der Erfindung, bei dem die Injektionsdosis vom Benutzer in einfacher Weise verstellt werden kann, und bei dem besondere Maßnahmen getroffen sind, um eine exakte Einstellung auch kleiner Injektionsdosen zu ermöglichen. Kleine Dosen, z.B. bei Insulin 1 Einheit, erfordern nämlich bei den handelsüblichen Kartuschen sehr kleine Verstellwege, z.B. bei einem bekannten Produkt einen Weg des Kolbens in dieser Kartusche von nur 0,27 mm. Die Erfindung ermöglicht es, auch solche kleinen Dosiermengen präzise einzustellen und zu injizieren.

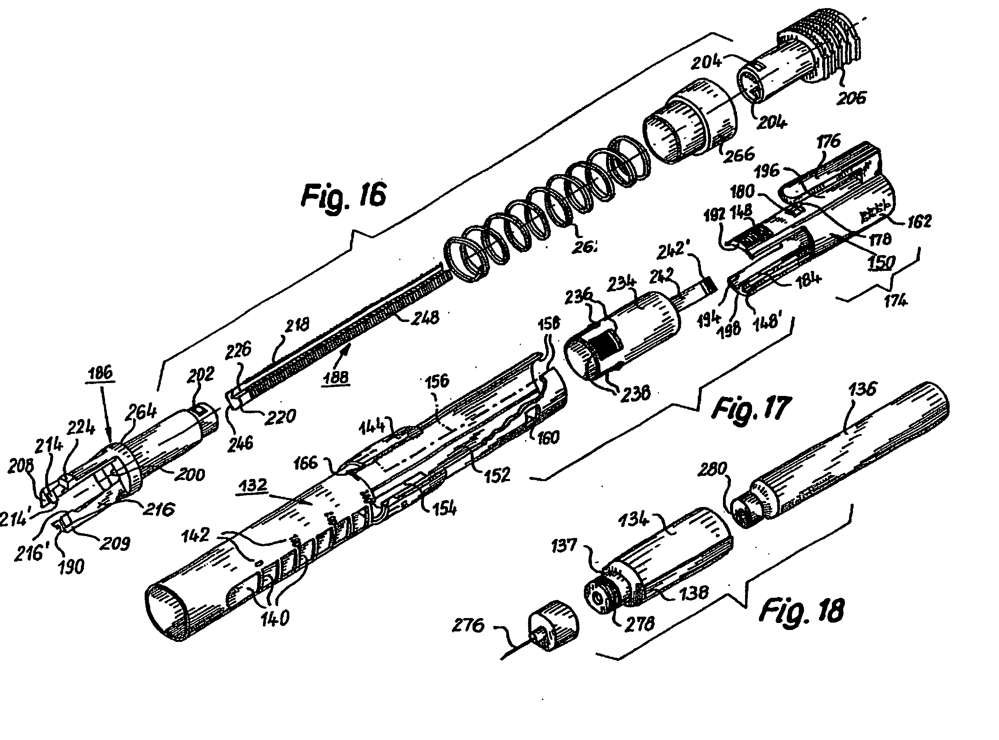

Bei diesem Ausführungsbeispiel weist das Gehäuse des Injektionsgeräts 130 mehrere Teile auf, und als Hauptteil ein rohrförmiges Gehäuseteil 132, dessen Gestalt am besten aus Fig. 17 hervorgeht, wobei in Fig. 17 rechts dieses Gehäuseteil 132 partiell weggeschnitten dargestellt ist, um einen Blick in sein Inneres zu gestatten.

Das Gehäuseteil 132 dient dazu, im Inneren seines proximalen Abschnitts einen durchsichtigen Halter 134 (Fig. 18) für eine ebenfalls durchsichtige Kartusche 136 üblicher Bauart zu fuhren. Dazu ist das Gehäuseteil 132 innen mit (nicht dargestellten) Führungsstegen versehen, welche längliche Vorsprünge 137, 138 des Halters 134 in Längsrichtung führen, so daß sich dieser Halter im proximalen Abschnitt des rohrförmigen Gehäuseteils 132 nicht verdrehen kann. Dieser proximale Abschnitt ist auch auf beiden Selten mit Fenstern 140 versehen, durch welche man nachschauen kann, wieviel Flüssigkeit sich noch in der Kartusche 136 befindet. Zahlen 142 an diesen Fenstern gestatten eine grobe Schätzung des restlichen Inhalts der Kartusche 136. Die Fig. 14 und 17 zeigen nur die Fenster 140 auf einer Seite des rohrförmigen Gehäuseteils 132; diesen liegen symmetrisch auf der Gegenseite entsprechende Fenster gegenüber.

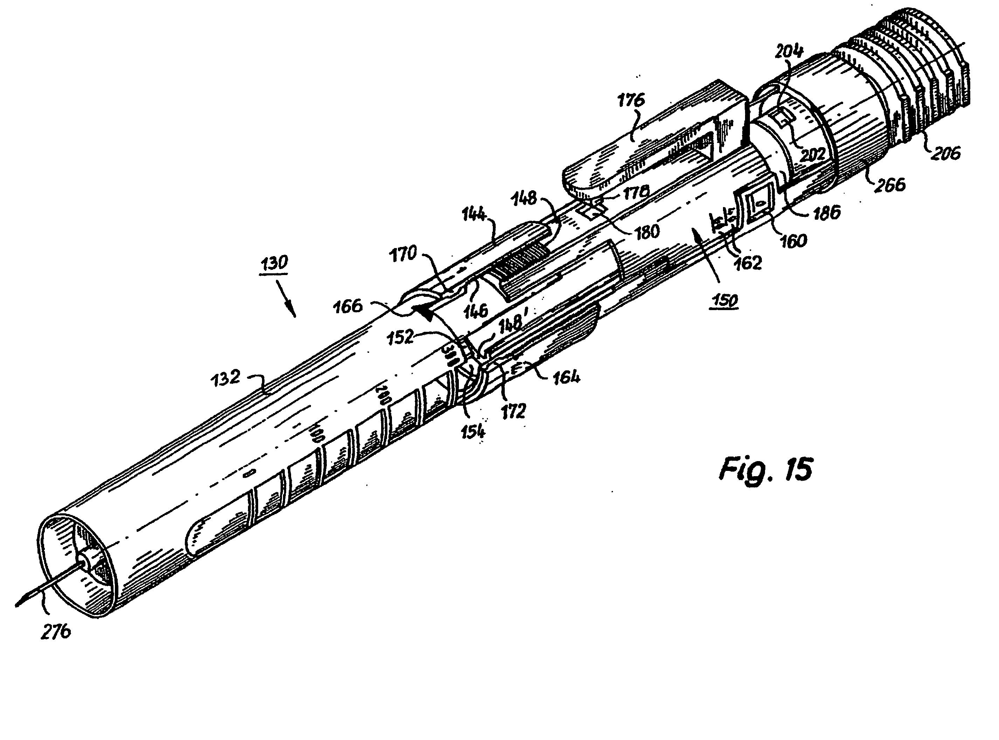

Etwa in der Mitte des rohrförmigen Gehäuseteils 132 ist auf diesem eine Gewindehülse 144 drehbar, aber axial unverschieblich, befestigt. Wie Fig. 15 am besten zeigt, hat die Hülse 144 auf ihrer Innenseite ein Innengewinde 146 in Gestalt eines Feingewindes, und dieses steht in Eingriff mit einem entsprechenden Außengewinde 148 eines zwecks Dosiseinstellung im Gehäuseteil 132 längsverschiebbaren Gehäuseteils 150, dessen Form am besten aus Fig. 17 hervorgeht.

Gemäß Fig. 17 hat das rohrförmige Gehäuseteil 132 in seinem distalen Bereich unten (bezogen auf Fig. 17) eine relativ breite Längsnut 152, die etwa in der Mitte des Gehäuseteils 132 ein rechteckförmiges Fenster 154 aufweist, dessen Form aus Fig. 17 klar ersichtlich ist. Durch dieses Fenster 154 ragt das Außengewinde 148' (Fig. 17) des Gehäuseteils 150 - durch die Wand des rohrförmigen Gehäuseteils 132-radial nach außen, damit es mit dem Innengewinde 146 in Eingriff kommt.

Wie ferner in Fig. 17 - teilweise nur mit strichpunktierten Linien 156 - angedeutet, hat das rohrförmige Gehäuseteil 132 auf seiner distalen Seite einen Längsschlitz 158, der sich vom distalen Ende dieses Gehäuseteils bis fast zu dessen Mitte erstreckt. Der Längsschlitz 158 liegt dem Fenster 154 diametral gegenüber und ist gleich breit wie dieses. Durch den Längsschlitz 158 ragt das Außengewinde 148 des Gehäuseteils 150 radial nach außen, zwecks Eingriff mit dem Innengewinde 146 der Gewindehülse 144.

Das rohrförmige Gehäuseteil 132 hat ferner ein Sichtfenster 160, durch welches eine (grobe) Dosisanzeige 162 auf dem Gehäuseteil 150 abgelesen werden kann, vgl. Fig. 15. Die Feinanzeige der Dosis, z.B. von "0" bis "19", befindet sich in Form von Indicia 164 auf der Gewindehülse 144, und auf dem rohrförmigen Gehäuseteil 132 befindet sich ein zugeordneter Anzeigepfeil 166. Der Benutzer addiert also den Wert 162 im Fenster 160, z.B. "0", zum Wert 164, auf den der Pfeil 166 zeigt, um die augenblickliche Einstellung des Injektionsgeräts 130 zu erhalten, die in Fig. 4 Null beträgt. Dreht er dann die Hülse 144 in Richtung des Pfeiles 168 (Fig. 14) um drei Teilstriche weiter, so hat er eine Dosis von drei Einheiten eingestellt; diese Einstellung muß er nur dann verändern, wenn er anschließend eine andere Dosis injizieren will.

Beträgt die gewünschte Dosis beispielsweise stets 3 Einheiten, so kann die Gewindehülse 144 in dieser Stellung verbleiben. Das ist insbesondere für Diabetiker mit Sehschwäche von Vorteil, da diese dann vor einer Injektion keine Änderung der Einstellung der Gewindehülse 144 vornehmen müssen. Vielmehr muß ein solcher Patient dann lediglich das Gerät spannen, auf die gewünschte Körperstelle aufsetzen, und dann die Injektion auslösen, ohne sich um die Dosis zu kümmern.

Soll die Dosis geändert werden, so muß die Gewindehülse 144 entsprechend verdreht werden, wobei eine Drehung in Richtung des Pfeiles 164 eine Erhöhung der Injektionsdosis und eine Drehung entgegen dem Pfeil 164 eine Reduzierung der Injektionsdosis bewirkt.

Die Gewindehülse 144 ist, wie in Fig. 15 dargestellt, auf ihrer Innenseite mit einem ringförmigen Vorsprung (Bund) 170 versehen, der in eine zu ihm komplementäre Ringnut 172 auf der Außenseite des rohrförmigen Gehäuseteils 132 eingreift. Wie dargestellt, haben der Bund 170 und die Ringnut 172 schräge Flanken, so daß die Gewindehülse 144 einfach auf das Gehäuseteil 132 aufgepreßt werden kann, bis sie mit dem Bund 170 in die Ringnut 172 einrastet. Dadurch kann die Gewindehülse 144 nach ihrer Montage auf dem Gehäuseteil 132 zwar auf diesem verdreht, aber nicht axial relativ zu ihm verschoben werden.

Das Innengewinde 146 der Gewindehülse 144 ist so lang, daß es das Fenster 154 (Fig. 17) überdeckt.

Das Gehäuseteil 150 ist im rohrförmigen Gehäuseteil 132 linear verschiebbar, aber nicht verdrehbar; beide Teile bilden zusammen das Gehäuse des Injektionsgeräts. Der distale Abschnitt 174 (Fig. 17) des Gehäuseteils 150 hat im wesentlichen die Form eines Rohres mit kreiszylindrischem Querschnitt, an dessen distalem Ende ein Clip 176 befestigt ist, der mit einem Vorsprung 178 zum Eingriff in eine radiale Rastöffnung 180 des Gehäuseteils 150 dient. Wird das Gehäuseteil 150 linear relativ zum Gehäuseteil 132 verschoben (durch Verdrehung der Gewindehülse 144), so verschiebt sich die Lage der Rastöffnung 180 relativ zum Gehäuseteil 132, und dies ermöglicht eine Verstellung der Injektionsdosis: Je mehr die Rastöffnung 180 in distaler Richtung verschoben wird, umso größer wird die Injektionsdosis.

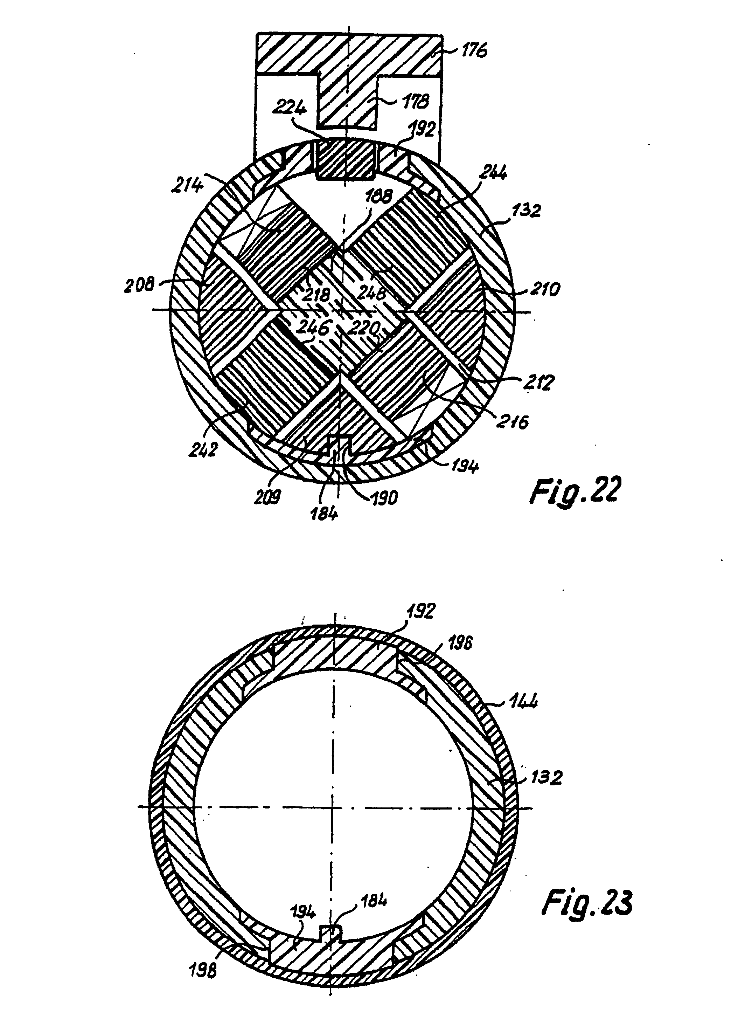

Wie Fig. 17 und 23 am besten zeigen, hat das Gehäuseteil 150 In seinem Inneren einen axial verlaufenden Führungsvorsprung 184, der zur Führung eines Teils 186 (Fig. 16) dient, das im folgenden als Zustellteil bezeichnet wird, weil es bei einer Zahnstange 188 (Fig. 16, 30, 31), die in diesem Zustellteil geführt ist, eine Zustellbewegung in proximaler Richtung bewirkt. Hierzu hat das Zustellteil 186 eine in axialer Richtung verlaufende Nut 190 (Fig. 16), in welche der Führungsvorsprung 184 eingreift.

Auf seiner proximalen Seite hat das Gehäuseteil 150 zwei einander diametral gegenüberliegende, axiale Vorsprünge 192, 194, die bei der Montage in Richtung zueinander federnd auslenkbar sind. Der in Fig. 17 obere Vorsprung 192 trägt den Gewindeabschnitt 148, und der untere Abschnitt 194 den Gewindeabschnitt 148'. Auf dem oberen Vorsprung 192 verläuft eine längliche Verbreiterung 196, die nach der Montage vom Längsschlitz 158 des Gehäuseteils 132 geführt wird, und auf dem unteren Vorsprung 194 verläuft eine längliche Verbreiterung 198, die nach der Montage in der Nut 152 des Gehäuseteils 132 geführt ist. Dadurch ist das Gehäuseteil 150 im montierten Zustand, wie er in den Fig. 14 und 15 dargestellt ist, in Längsrichtung im Gehäuseteil 132 geführt und mit diesem dadurch verbunden, daß das Innengewinde 146 der Gewindehülse 144 in die Außengewindeabschnitte 148 und 148' des Gehäuseteils 150 eingreift.

Wie Fig. 16 zeigt, hat das Zustellteil 186 im Bereich seiner Längsachse eine Ausnehmung 200 mit quadratischem Querschnitt, in welcher die Zahnstange 188 längsverschiebbar geführt ist. Diese hat ebenfalls einen im wesentlichen quadratischen Querschnitt, wie sich das aus den Fig. 22, 30 und 31 ergibt. Das Zustellteil 186 hat auf seiner distalen Seite oben und unten einen Rastvorsprung 202, welche Rastvorsprünge bei der Montage in entsprechende Rastausnehmungen 204 eines Betätigungsknopfes 206 einrasten. (Das Zustellteil 186 ist in Fig. 15 teilweise weggeschnitten dargestellt, d.h. man sieht nur dessen distales Ende.)

Das Zustellteil 186 hat drei Führungsteile 208, 209, 210, von denen das Führungsteil 209 mit der Längsnut 190 versehen ist, vgl. Fig. 22. (In Fig. 16 ist das Führungsteil 210 - zwecks besserer Darstellung-nicht gezeigt.) Diese Führungsteile gleiten längs der Innenwand 212 des rohrförmigen Gehäuseteils 132 und führen dadurch das Zustellteil 186 in diesem.

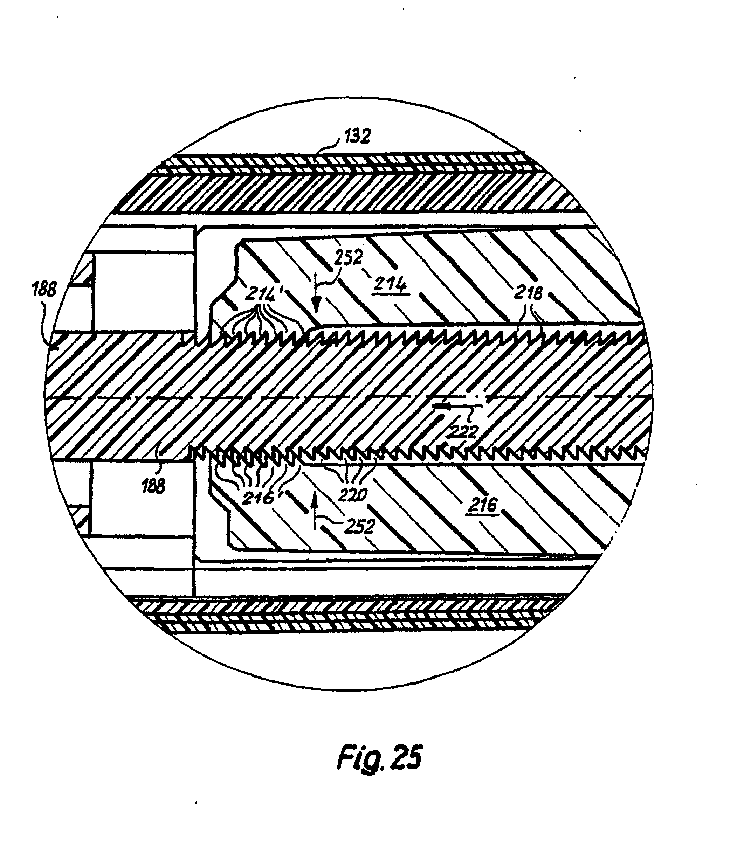

Ferner enthält das Zustellteil 186 zwei zangenartig ausgebildete, elastische Eingriffsglieder 214, 216, die an ihren freien Enden jeweils mit sieben Rastzähnen 214', 216' versehen sind. Diese Rastzähne sind sehr klein und deshalb nur in der Vergrößerung der Fig. 25 gut darstellbar.

Die Rastzähne 214', 216' liegen einander ohne axiale Versetzung direkt gegenüber, wie das Fig. 25 zeigt, während die ihnen entsprechenden Zahnungen 218 bzw. 220 auf den beiden hier wirksamen, gegenüberliegenden Seiten der Zahnstange 188 um eine halbe Zahnteilung gegeneinander versetzt sind. Deshalb greifen bei dieser Darstellung die Rastzähne 214' voll und mit Vorspannung in die Zähne 218 der Zahnstange 188 ein, während die Rastzähne 216' nur halb, und ebenfalls mit Vorspannung, im Eingriff mit den Zähnen 220 der Zahnstange 188 sind. (Naturgemäß können alternativ die Zähne auf der Zahnstange 188 einander ohne axiale Versetzung gegenüberliegen, und man würde dann die axiale Versetzung bei den Rastzähnen 214', 216' vorsehen, doch wird die dargestellte Form bevorzugt.)

Infolge der Form der Zähne kann sich die Zahnstange 188 ersichtlich nur in Richtung des Pfeiles 222 verschieben, also in proximaler Richtung, wobei die Eingriffsglieder 214, 216 elastisch radial nach außen ausgelenkt werden. Wird die Zahnstange 188 um eine halbe Zahnteilung in der Richtung 222 verschoben, so greifen die Zähne 216' voll In die Zähne 220 der Zahnstange 188 ein, und die Zähne 214' sind dann nur halb im Eingriff mit den Zähnen 218. Auf diese Weise kann ein Vorschub um eine halbe Zahnteilung realisiert werden, und eine halbe Zahnteilung entspricht hier beispielsweise 0,27 mm bzw. einer Einheit der zu injizierenden Flüssigkeit.

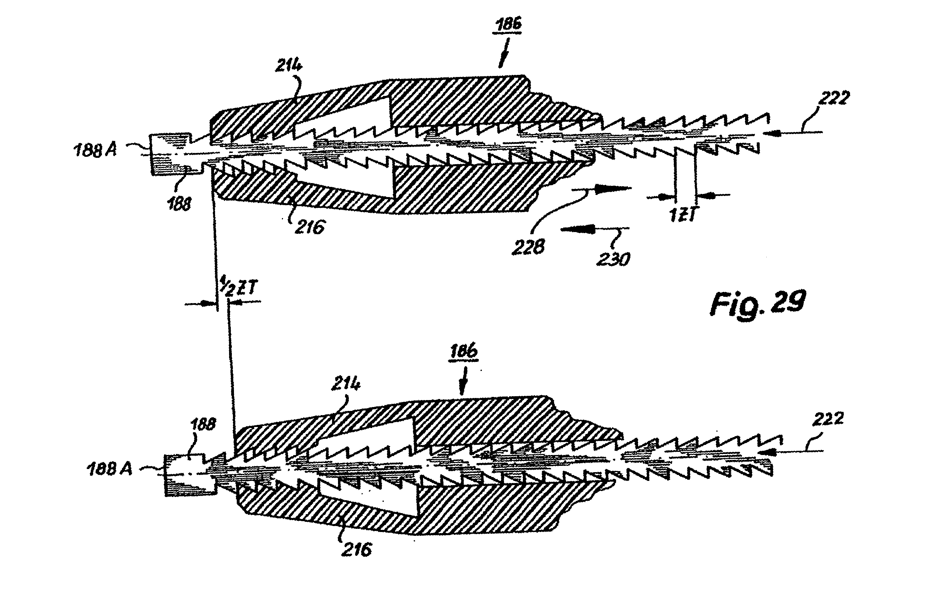

Fig. 29 zeigt in schematisierter Darstellung einen Vorschubvorgang der Zahnstange 222 um eine halbe Zahnteilung, was als "1/2 ZT" in der Zeichnung angegeben ist. Dies geschieht dadurch, daß das Zustellteil 186 zuerst in Richtung eines Pfeiles 228 in distaler Richtung um eine halbe Zahnteilung verschoben wird, wie im unteren Teil von Fig. 29 dargestellt, wobei die Zahnstange 188 in einer nachfolgend noch beschriebenen Weise festgehalten wird und die Zähne des Zustellteils 186 um eine halbe Zahnteilung weitergleiten, und daß das Zustellteil 186 anschließend in Richtung eines Pfeils 230 in proximaler Richtung verschoben wird, wodurch dann die Zahnstange 188 um eine halbe Zahnteilung in proximaler Richtung verschoben wird.

Am Zustellteil 186 ist ferner auch noch ein federndes Rastglied 224 vorgesehen, das beim Spannen des Injektionsgeräts 130 in die Rastöffnung 180 einrastet, und das durch Drücken auf den als Auslöser dienenden Clip 176 aus der Rastöffnung 180 herausgedrückt werden kann, um eine Injektion auszulösen, wie bei Fig. 8A und 9A ausführlich beschrieben. Wie aus Fig. 22 hervorgeht, liegt das Rastglied 224 im freien Raum zwischen zwei benachbarten Eingriffsgliedern 214, 244, und es liegt einer Kante der Zahnstange 188 gegenüber. Auf diese Weise gelingt es, das Injektionsgerät in raumsparender Weise aufzubauen, da für den Federweg des Rastglieds 224 auf diese Weise genügend Platz zur Verfügung steht.

Um die Zahnungen der Zahnstange 188 besser sichtbar zu machen, ist diese in Fig. 16, 30 und 31 mit einer Längsnut 226 dargestellt. Diese Längsnut 226 existiert in der Realität nicht, wie sich z.B. aus dem Schnitt der Fig. 22 klar ergibt. Sie dient lediglich zur besseren Veranschaulichung der Zahnungen.

Das Injektionsgerät 130 enthält ferner ein Halterteil 234, das in Fig. 17 in raumbildlicher Form dargestellt ist und das im montierten Zustand durch einen Rastvorgang mit dem Halter 134 (Fig. 18) verbunden ist. Das Halterteil 234 hat auf seiner Außenseite Vorsprünge 236, deren Funktion nachfolgend erläutert wird, und es hat auf seiner Außenseite Mikrorastungen 238, die mit entsprechenden Mikrorastungen 240 (Fig. 20, 21) auf der Innenseite des distalen Endes des Halters 134 zusammenwirken.

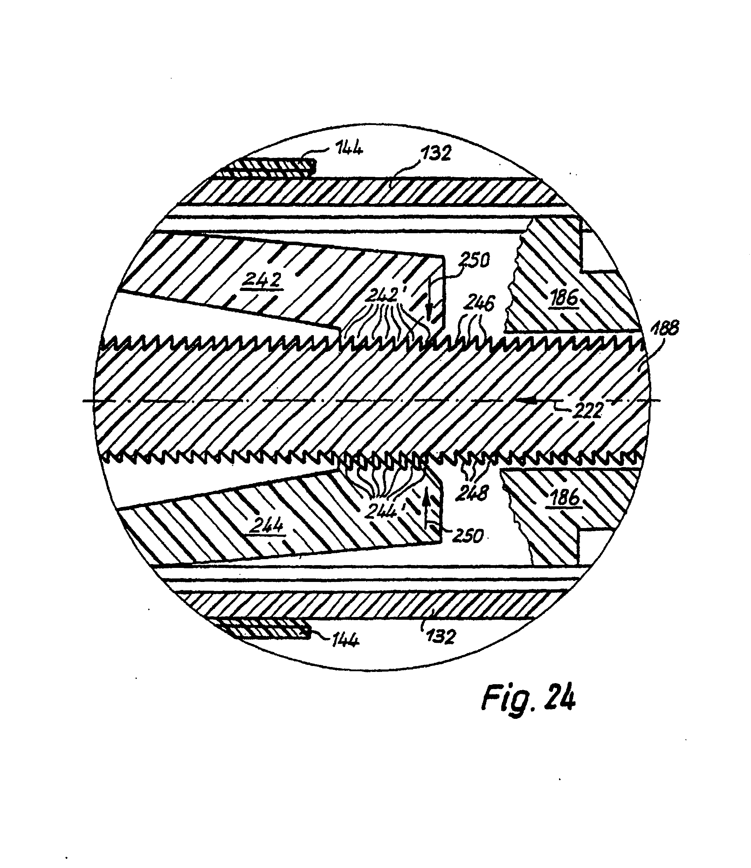

Das Halterteil 234 hat ferner, wie am besten aus Fig. 19 und 24 hervorgeht, an seinem distalen Ende zwei zangenartige, einander gegenüberliegende, elastisch auslenkbare Eingriffsglieder 242, 244. Die starke Vergrößerung gemäß Fig. 24 zeigt, daß diese Eingriffsglieder jeweils mit sieben Zähnen 242' bzw. 244 versehen sind, die einander ohne axiale Versetzungen gegenüberliegen, analog den Zähnen 214' und 216' der Fig. 25. (Im Prinzip genügt naturgemäß ein einziger Zahn, oder auch nur ein halber Zahn, doch wird eine größere Zahl von Zähnen bevorzugt.)

Die Zähne 242' und 244' greifen ein in entsprechende Zähne 246 bzw. 248 auf zwei gegenüberliegenden Seiten der Zahnstange 188. Diese Zähne 246, 248 sind relativ zueinander um eine halbe Zahnteilung versetzt, so daß immer nur entweder die Zähne 242' voll in die Zähne 246 eingreifen, wie in Fig. 24 dargestellt, oder umgekehrt nur die Zähne 244' voll in die Zähne 248 eingreifen.

Auch die Zähne 242', 244', 246 und 248 sind so ausgebildet, daß sie nur eine Verschiebung der Zahnstange 188 in proximaler Richtung (Pfeil 222) gestatten.

Die Eingriffsglieder 242, 244 sind so ausgebildet, daß sie mit Vorspannung gegen die Zahnstange 188 anliegen, wie in Fig. 24 durch Pfeile 250 angedeutet. Sie sind deshalb nach Art einer Spannzange ausgebildet.

Ebenso sind die Eingriffsglieder 214, 216 des Zustellteils 186 so ausgebildet, daß sie mit Vorspannung gegen die Zahnstange 188 anliegen, wie in Fig. 25 durch Pfeile 252 angedeutet.

Die Vorspannungen 250, 252 erhöhen die Reibung zwischen den betreffenden Eingriffsgliedern und der Zahnstange 188, was die Funktion verbessert und eine große Funktionssicherheit gewährleistet.

Der Umstand, daß die Vorspannungen 250, 252 jeweils symmetrisch auf die Zahnstange 188 wirken, verhindert, daß diese durch diese Kräfte verbogen wird.

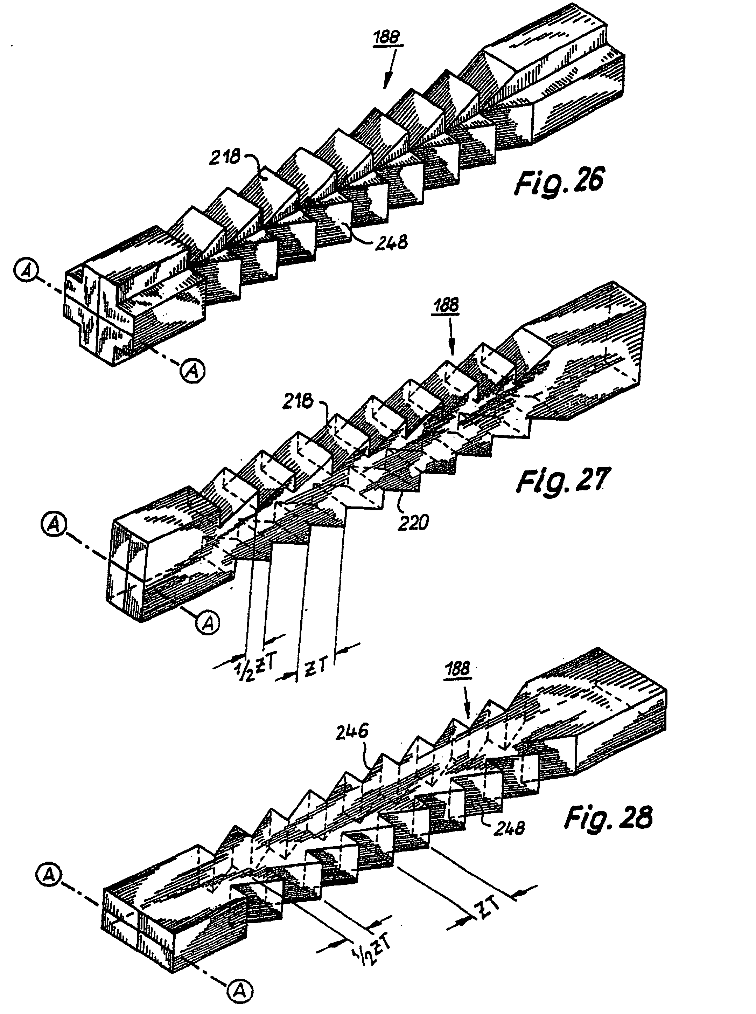

Das Verständnis der Struktur der Zahnstange 188 wird durch die Schemadarstellungen der Fig. 26 bis 28 erleichtert. In allen drei Figuren ist die Achse A-A dargestellt, um eine einfache Orientierung zu ermöglichen.

Fig. 26 zeigt die Zahnstange 188 raumbildlich und stark schematisiert, um das Verständnis zu erleichtern. Von den vier Zahnungen sieht man in Fig. 26 nur die beiden Zahnreihen 218 und 248, welche dieselbe Zahnteilung und dieselbe Phasenlage haben, d.h. ihre Täler liegen in derselben Ebene.

Fig. 27 zeigt dann nur die beiden gegenüberliegenden Zahnreihen 218 und 220, die mit dem Zustellteil 186 zusammenwirken und die um eine halbe Zahnteilung (1/2 ZT) gegeneinander versetzt sind, was man auch als Phasenverschiebung von 180° bezeichnen kann.

Fig. 28 zeigt nur die beiden gegenüberliegenden Zahnreihen 246 und 248, die ebenfalls um eine halbe Zahnteilung (1/2 ZT) gegeneinander versetzt sind, also ebenfalls eine Phasenverschiebung von 180° haben.

Die Zahnreihen 246 und 248 wirken mit dem Halterteil 234 (Fig. 17) zusammen und dienen zu dessen Antrieb, analog dem Antrieb des Teils 66 in Fig. 11A.

### Montage

Zunächst wird auf das rohrförmige Gehäuseteil 132 die Gewindehülse 144 in der bereits beschriebenen Weise aufgerastet. Dann wird das Teil 234 in das Gehäuseteil 132 von dessen distaler Seite aus eingeschoben. Das Teil 234 gleitet mit seinen Rastvorsprüngen 236 über einen entsprechenden inneren Rastwulst 260 des Gehäuseteils 132. Die Rastvorsprünge 236 sind so ausgebildet, daß sie eine solche Verschiebung in proximaler Richtung ermöglichen, nicht aber in distaler Richtung, da sie dann als Anschläge wirken, wie in Fig. 21 dargestellt. (In Fig. 21 ist der als Auslöser dienende Clip 176 um 45° verdreht gezeichnet, d.h. er wäre in dieser Schnittdarstellung eigentlich nicht sichtbar. Fig. 21 zeigt die gespannte Stellung für die Injektion von zwei Einheiten, entsprechend einer einzigen Zahnteilung der Zahnstange 188.)

Anschließend wird das Gehäuseteil 150 (Fig. 17) in das rohrförmige Gehäuseteil 132 eingeschoben, wobei das Gewinde 148' in das Fenster 154 im Gehäuseteil 132 einrastet, und durch Drehen der Gewindehülse 144 wird das Gehäuseteil 150 in seine Nullstellung gebracht, die im Fenster 160 angezeigt wird. Damit ist das Gehäuse fertig aus den Teilen 132, 144 und 150 zusammengesetzt und hat die Form, die aus Fig. 14 hervorgeht. Dies entspricht der Nullstellung, also der Stellung für die Injektion von 0 Einheiten.

Nun führt man von der distalen Seite dieses Gehäuses das Zustellteil 186 in das Gehäuse ein. In die Öffnung 200 des Zustellteils 186 steckt man die Zahnstange 188 so weit ein, daß die Eingriffsglieder 214, 216 des Zustellteils 186 in die ersten Zähne am proximalen Ende der Zahnstange 188 einrasten. Dies entspricht etwa der Stellung gemäß Fig. 25, aber noch um einen Zahn weiter nach links verschoben.

Anschließend setzt man eine Spannfeder 262 (Fig. 16) vom distalen Ende in das rohrförmige Gehäuseteil 132 ein, und zwar bis zum Anschlag gegen den Bund 264 des Zustellteils 186, wobei das Zustellteil 186 zweckmäßig mit seinem federnd auslenkbaren Rastvorsprung 224 In der Rastöffnung 180 verrastet wird. Dann wird ein Bundteil 266 durch Rasten oder Kleben im distalen Ende des rohrförmigen Gehäuseteils 132 befestigt. Es dient als Widerlager für die Spannfeder 262.

In den Fig. 19 bis 21 ist nur der Ringraum 270 dargestellt, in welchem sich die Spannfeder 262 nach dem Einbau befindet, da diese Zeichnungen sehr unübersichtlich geworden wären, wenn auch die Spannfeder 262 dort dargestellt würde. Bis auf die Spannfeder 262 können alle Teile des Injektionsgeräts 130 aus einem geeigneten Kunststoff hergestellt werden.

Nach der Befestigung des Bundteils 266 wird der Betätigungsknopf 206 am distalen Ende des Zustellteils 186 befestigt, wie bereits beschrieben (Teile 202, 204 in Fig. 16).

Die Mechanik ist nun weitgehend fertig montiert, und das proximale Ende 188A (Fig. 29) der Zahnstange 188 steht zur Betätigung des Kolbens 272 (Fig. 19 bis 21) der Kartusche 136 bereit. Die Eingriffsglieder 214, 216 des Zustellteils 186 und die Eingriffsglieder 242, 244 des Halterteils 234 greifen dabei interdigital ineinander, wie sich das aus der Schnittdarstellung der Fig. 22 klar ergibt. Dies ist sehr ähnlich Fig. 11A, läßt sich aber in einem Längsschnitt nicht darstellen. Ein Vergleich der Fig. 19 und 20 zeigt dieses Ineinandergreifen.

Man löst nun durch Druck auf den Auslöser 176 die Verrastung mit dem Rastglied 224 aus, wodurch - durch die Wirkung der Feder 262-die Zahnstange 188 die Lage gemäß Fig. 19 und 20 einnimmt, und führt dann vom proximalen Ende her die Kartusche 136 in das rohrförmige Gehäuseteil 132 ein, bis ihr Kolben 272 gegen das proximale Ende 188A der Zahnstange 188 anliegt. Anschließend führt man, ebenfalls vom proximalen Ende des Gehäuseteils 132 her, den Halter 134 in dieses Gehäuseteil ein. Durch eine entsprechende Kraft wird dessen innere Mikrorastung 240 zur Verrastung gebracht mit der Mikrorastung 238 auf dem Halterteil 234. Hierbei wird die Kraft gemessen, die für diese Verrastung notwendig ist. Wenn diese Kraft ansteigt, weil der Kolben 272 der Kartusche 136 zur Anlage gegen das proximale Ende 188A der Zahnstange 188 kommt, wird die Verrastung beendet, da sich dann der Halter 134 in der richtigen Stellung auf dem Halterteil 234 befindet.

Das Gerät ist nun fertig, und durch Aufschrauben einer Nadel 276 auf ein Gewinde 278 am proximalen Ende des Halters 134 kann es vom Patienten benutzungsbereit gemacht werden. Diese Nadel 276 durchdringt dabei mit ihrem distalen Ende eine Gummimembran 280 (Fig. 18) am proximalen Ende der Kartusche 136. (Die Nadel 276 sollte nach jeder Injektion gewechselt werden. Sie wird erst vor einer Injektion befestigt und wird bis dahin in einem sterilen Behälter aufbewahrt.)

### Arbeitsweise

Vor einer Injektion stellt man durch Drehen an der Gewindehülse 144 die gewünschte Dosis ein, z.B. 2 Einheiten, wie in Fig. 21 dargestellt. Dadurch wird das Gehäuseteil 150 mit seiner Rastöffnung 180 entsprechend weit in distaler Richtung relativ zum Gehäuseteil 132 verschoben. z.B. bei 2 Einheiten um eine Zahnteilung der Zahnstange 188, entsprechend beispielsweise 0,54 mm. Deshalb ist dies in Fig. 21 zeichnerisch nicht darstellbar.

Anschließend wird durch Ziehen in distaler Richtung am Betätigungsknopf 206 die Spannfeder 262 (Fig. 16) gespannt, und der Rastvorsprung 224 wird zum Einrasten in die Rastöffnung 180 gebracht.

Bei dieser Spannbewegung wird durch die Eingriffsglieder 242, 244 (Fig. 19), die wie Spannbacken wirken und den Halter 134 mitnehmen, die Nadel 276 ins rohrförmige Gehäuseteil 132 hineingezogen, vgl. Fig. 21. Dabei gelangen die Vorsprünge 236 des Halterteils 234 (am besten sichtbar in Fig. 17) zum Anschlag gegen den Bund 260 im Gehäuseteil 132, und wenn dies der Fall ist, gleiten die Zähne 214', 216' um die eingestellte eine Zahnteilung über die Zähne 218, 220 der Zahnstange 188 hinweg und stellen so die Dosis ein, wobei anschließend das Rastglied 224 gemäß Fig. 21 in die Ausnehmung 180 einrastet.

Nach dem Auslösen mittels des Auslösers 176 verschiebt die gespannte Feder 262 über die Eingriffsglieder 214, 216 die Zahnstange 188 in proximaler Richtung, und durch die wie Klemmbacken wirksamen Eingriffsglieder 242, 244 des Halterteils 234 wird diese Bewegung direkt auf die Nadel 276 übertragen, so daß diese in das Gewebe des Patienten einsticht. Dabei kommt das Halterteil 234 mit einem radialen Vorsprung 284 (Fig. 20) zum Anschlag gegen den Ringbund 260 im Inneren des Gehäuseteils 132 und begrenzt so die Einstichtiefe der Nadel 276.

Da aber die Zahnstange 188 ihre Bewegung in proximaler Richtung (Pfeil 222 in Fig. 24) noch fortsetzt, gleitet sie (bei diesem Beispiel, bei dem eine Zahnteilung als Injektionsdosis eingestellt wurde) in Fig. 24 relativ zu den Eingriffsgliedern 242, 244 in Richtung des Pfeiles 222 um eine Zahnteilung weiter, wodurch die entsprechende Flüssigkeitsmenge (2 Einheiten) durch Verschiebung des Kolbens 272 aus der Kartusche 136 ausgepreßt wird. Ersichtlich ist in gleicher Weise eine Verschiebung auch nur um eine halbe Zahnteilung, 1,5 Zahnteilungen, etc. möglich, und dies wird dadurch erreicht, daß die Anordnungen nach Fig. 24 und 25 im Prinzip gleich ausgebildet sind.

Auf diese Weise ist beim Ausführungsbeispiel eine Dosiseinstellung von 1 ... 60 Einheiten möglich, was den Bedarf der Praxis abdeckt.

Naturgemäß sind im Rahmen der vorliegenden Erfindung vielfache Abwandlungen und Modifikationen möglich. So könnte man z.B. statt der Versetzung der Zähne um eine halbe Zahnteilung, wie sie in den Fig. 24 bis 29 dargestellt ist, eine Anordnung mit drei Zahnreihen verwenden, von denen jede nur um 1/3 Zahnteilung gegen die andere versetzt ist, wobei jeder Zahnreihe dann ein entsprechendes Eingriffsglied zuzuordnen ist. Solche und andere Modifikationen ergeben sich für den Fachmann ohne Schwierigkeiten und liegen im Rahmen der Erfindung.

## Patentansprüche

1. Injektionsgerät für die Abgabe mehrerer Injektionen, welches Injektionsgerät ein Gehäuse (132, 150) zur Aufnahme eines Vorratsbehälters (136) für eine zu injizierende Flüssigkeit und eine mittels eines Betätigungsgliedes axial verschiebbare Zahnstange (188) zum Auspressen von Flüssigkeit aus einem solchen im Gehäuse (132, 150) angeordneten Vorratsbehälter (136) aufweist, welche Zahnstange (188) mindestens zwei Zahnreihen (218, 220, 246, 248) aufweist,
**gekennzeichnet dadurch,**
**dass** die Zahnreihen mit einem mit der Zahnstange (188) zusammenwirkenden, axial verschiebbaren Gegenglied (186), insbesondere dem Betätigungsglied, zusammenwirken, welches Gegenglied mindestens zwei erste Eingriffsglieder (214, 216) aufweist, von denen jedes einer der mindestens zwei Zahnreihen (218, 220, 246, 248) zugeordnet und mit mindestens einem Eingriffselement (214', 216') versehen ist und über dieses in federndem Eingriff mit der zugeordneten Zahnreihe steht,
wobei jeweils ein Eingriffsglied über sein mindestens eines Eingriffselement (214', 216') mit der ihm zugeordneten Zahnreihe (218, 220) in vollem Eingriff steht,
und mindestens ein anderes Eingriffsglied über sein mindestens eines Eingriffselement mit der ihm zugeordneten Zahnreihe (218, 220) nicht in vollem Eingriff steht.

2. Injektionsgerät nach Anspruch 1, bei welchem die mit dem Gegenglied zusammenwirkenden Zahnreihen (218, 220) der Zahnstange (188) im wesentlichen symmetrisch zur Längsachse der Zahnstange (188) angeordnet sind.

3. Injektionsgerät nach Anspruch 1 oder 2, bei welchem das Gegenglied als Antriebsorgan (186) zur Verschiebung des Behälters (136) im Gehäuse (132, 150) des Injektionsgeräts ausgebildet ist.

4. Injektionsgerät nach einem oder mehreren der Ansprüche 1 bis 3, bei welchem die Zahnstange (188) einen rechteckförmigen, insbesondere quadratischen Querschnitt aufweist,
und das Gegenglied zwei Eingriffsglieder (214, 216) aufweist, die mit Zahnreihen (218, 220) auf einander gegenüberliegenden Seiten dieser Zahnstange zusammenwirken.

5. Injektionsgerät nach einem der vorhergehenden Ansprüche, bei welchem zusätzlich zu den am axial verschiebbaren Betätigungsglied vorgesehenen ersten Eingriffsgliedern (214, 216) zweite Eingriffsglieder (242, 244) vorgesehen sind, welche ebenfalls über Eingriffselemente (242', 244') mit der Zahnstange (188) in federndem Eingriff stehen,
wobei die zweiten Eingriffsglieder (242, 244) dazu ausgebildet sind, im Verlauf einer distalen Bewegung des Betätigungsglieds die Zahnstange (188) in einer vorgegebenen Stellung zu arretieren.

6. Injektionsgerät nach Anspruch 5, bei welchem die zweiten Eingriffsglieder (242, 244) dazu ausgebildet sind, im Verlauf einer proximalen Bewegung des Betätigungsglieds eine Bewegung der Zahnstange (188) in proximaler Richtung zu ermöglichen.

7. Injektionsgerät nach Anspruch 5 oder 6, bei welchem die zweiten Eingriffsglieder (242, 244) so ausgebildet sind, dass sie einen vollen Eingriff zwischen einem der zweiten Eingriffsglieder und der Zahnstange (188) jeweils schon nach einer vorgegebenen Verschiebung der Zahnstange (188) ermöglichen, die weniger groß ist als eine Zahnteilung (ZT) der Zahnstange (188).

8. Injektionsgerät nach einem der Ansprüche 5 bis 7, bei welchem die Zahnstange (188) einen rechteckförmigen und bevorzugt quadratischen Querschnitt aufweist,
und auf zwei ersten einander gegenüberliegenden Seiten mit Zahnreihen (218, 220) für die ersten Eingriffsglieder (214, 216) und auf den beiden anderen einander gegenüberliegenden Seiten mit Zahnreihen (246, 248) für die zweiten Eingriffsglieder (242, 244) versehen ist.

9. Injektionsgerät nach Anspruch 8, bei welchem die ersten Eingriffsglieder (214, 216) und die zweiten Eingriffsglieder (242, 244) interdigital ineinandergreifen.

10. Injektionsgerät nach einem der Ansprüche 5 bis 9, bei welchem die zweiten Eingriffsglieder (242, 244) mit einem verschiebbaren Behälter (136) für Injektionsflüssigkeit, oder einer Aufnahme (134, 234) für einen solchen Behälter, verbunden sind.

11. Injektionsgerät nach einem der vorhergehenden Ansprüche, bei welchem die Eingriffselemente (214', 216', 242', 244') jeweils im wesentlichen komplementär zu den Zähnen der ihnen zugeordneten Zahnreihe (218, 220, 246, 248) ausgebildet sind.

12. Injektionsgerät nach einem der vorhergehenden Ansprüche, bei welchem zur Aufnahme des Vorratsbehälters (136) für die zu injizierende Flüssigkeit ein Halter (54; 134, 234) vorgesehen ist, dessen Länge veränderbar ist.

13. Injektionsgerät nach Anspruch 12, bei welchem der Halter (54; 134, 234) einen proximalen Abschnitt (56; 134) und einen distalen Abschnitt (66; 234) aufweist, welche durch eine verstellbare Verbindung (64; 236, 240) miteinander verbunden sind.

14. Injektionsgerät nach Anspruch 13, bei welchem die verstellbare Verbindung mittels einer axialen Kraft (Fig. 8A: K) verkürzbar ist, um die Gesamtlänge des Halters (54; 134, 234) zu beeinflussen.

15. Injektionsgerät nach Anspruch 13 oder 14, bei welchem die verstellbare Verbindung als Mikrorastung (64; 236, 240) ausgebildet ist.

16. Injektionsgerät nach einem der Ansprüche 5 bis 10 in Verbindung mit einem der Ansprüche 12 bis 15, bei welchem der hinsichtlich seiner Länge verstellbare Halter (134, 234) mit den zweiten Eingriffsgliedern (242, 244) fest verbunden ist.

17. Injektionsgerät nach einem der Ansprüche 12 bis 16, bei welchem der Halter (134, 234) im Gehäuse (132) zwischen einer proximalen und einer distalen Endstellung verschiebbar ist.

18. Injektionsgerät nach einem der Ansprüche 1 bis 11, bei welchem zur Aufnahme des Vorratsbehälters (136) für die zu injizierende Flüssigkeit ein Halter (54; 134, 234) mit einem proximalen Abschnitt (56; 134) und einem distalen Abschnitt (66; 234) vorgesehen ist,
welche Abschnitte durch eine Rastverbindung (64; 236, 240) miteinander verbunden sind.

19. Injektionsgerät nach Anspruch 18, bei welchem die Rastverbindung (64; 236, 240) eine Mehrzahl von möglichen Raststellungen aufweist.

20. Injektionsgerät nach Anspruch 18 oder 19 in Verbindung mit einem der Ansprüche 5 bis 10, bei welchem der distale Abschnitt (234) des Halters (134, 234) mit den zweiten Eingriffsgliedern (242, 244) fest verbunden ist.

## Claims

1. Injection device for administering a plurality of injections, which injection device comprises a housing (132, 150) for receiving a storage container (136) for a liquid to be injected, and a toothed rod (188) displaceable axially by means of an actuating member for expressing liquid from such a storage container (136) arranged in the housing (132, 150), which toothed rod (188) comprises at least two rows of teeth (218, 220, 246, 248), **characterised in that** the rows of teeth co-operate with an axially displaceable counterpart member (186), in particular the actuating member, which cooperates with the toothed rod (188), which counterpart member comprises at least two first engagement members (214, 216), each of which has associated therewith one of the at least two rows of teeth (218, 220, 246, 248) and is provided with at least one engagement element (214', 216') and through this is in resilient engagement with the associated row of teeth, in each case one engagement member being in full engagement with the row of teeth (218, 220) associated with it by means of its at least one engagement element (214', 216'), and at least one other engagement member not being in full engagement with the row of teeth (218, 220) associated with it by means of its at least one engagement element.

2. Injection device according to claim 1, wherein the rows of teeth (218, 220) of the toothed rod (188) cooperating with the counterpart member are arranged essentially symmetrically to the longitudinal axis of the toothed rod (188).

3. Injection device according to claim 1 or 2, wherein the counterpart member is embodied as a drive member (186) for displacing the container (136) in the housing (132, 150) of the injection device.

4. Injection device according to one or more of claims 1 to 3, wherein the toothed rod (188) comprises a rectangular, in particular square cross-section, and the counterpart member comprises two engagement members (214, 216) which co-operate with rows of teeth (218, 220) on opposing sides of this toothed rod.

5. Injection device according to one of the preceding claims, wherein in addition to the first engagement members (214, 216) provided on the axially displaceable actuating member, second engagement members (242, 244) are provided which also are in resilient engagement with the toothed rod (188) by means of engagement elements (242', 244'), the second engagement members (242, 244) being designed in order to immobilise the toothed rod (188) in a predetermined position in the course of a distal movement of the actuating member.

6. Injection device according to claim 5, wherein the second engagement members (242, 244) are designed in order to allow a movement of the toothed rod (188) in the proximal direction in the course of a proximal movement of the actuating member.

7. Injection device according to claim 5 or 6, wherein the second engagement members (242, 244) are designed so that they allow full engagement between one of the second engagement members and the toothed rod (188) in each case after a predetermined displacement of the toothed rod (188) which is less than one tooth division (ZT) of the toothed rod (188).

8. Injection device according to one of claims 5 to 7, wherein the toothed rod (188) comprises a rectangular and preferably square cross-section, and is provided on two first opposing sides with rows of teeth (218, 220) for the first engagement members (214, 216) and on the two other opposing sides with rows of teeth (246, 248) for the second engagement members (242, 244).

9. Injection device according to claim 8, wherein the first engagement members (214, 216) and the second engagement members (242, 244) engage in one another interdigitally.

10. Injection device according to one of claims 5 to 9, wherein the second engagement members (242, 244) are connected with a displaceable container (136) for injection liquid, or a receiving part (134, 234) for such a container.

11. Injection device according to one of the preceding claims, wherein the engagement elements (214', 216', 242', 244') are in each case essentially embodied complementary to the teeth of the row of teeth (218, 220, 246, 248) associated with them.

12. Injection device according to one of the preceding claims, wherein a holder (54; 134, 234) of variable length is provided for receiving the storage container (136) for the liquid to be injected.

13. Injection device according to claim 12, wherein the holder (54; 134, 234) comprises a proximal portion (56; 134) and a distal portion (66; 234) which are connected to one another by an adjustable connection (64; 236, 240).

14. Injection device according to claim 13, wherein the adjustable connection can be shortened by means of an axial force (fig. 8A: K) in order to influence the overall length of the holder (54; 134, 234).

15. Injection device according to claim 13 or 14, wherein the adjustable connection is embodied as a microdetent (64; 236, 240).

16. Injection device according to one of claims 5 to 10 in combination with one of claims 12 to 15, wherein the holder (134, 234) adjustable with regard to its length is connected firmly to the second engagement members (242, 244).

17. Injection device according to one of claims 12 to 16, wherein the holder (134, 234) is displaceable in the housing (132) between a proximal and a distal end position.

18. Injection device according to one of claims 1 to 11, wherein a holder (54; 134, 234) is provided with a proximal portion (56; 134) and a distal portion (66; 234) for receiving the storage container (136) for the liquid to be injected, which portions are connected to one another by a detent connection (64; 236, 240).

19. Injection device according to claim 18, wherein the detent connection (64; 236, 240) comprises a plurality of possible detent positions.

20. Injection device according to claim 18 or 19 in combination with one of claims 5 to 10, wherein the distal portion (234) of the holder (134, 234) is connected firmly to the second engagement members (242, 244).

## Revendications

1. Dispositif d'injection qui est destiné à délivrer plusieurs injections, comprenant un boîtier (132, 150) pour la réception d'un réservoir (136) pour un liquide à injecter et une crémaillère (188) pouvant être déplacée axialement par l'intermédiaire d'un actionneur pour éjecter le liquide hors d'un tel réservoir (136) disposé dans le boîtier (132, 150), ladite crémaillère (188) présentant au moins deux rangées de dents (218, 220, 246, 248),
**caractérisé en ce que**
les rangées de dents coopèrent avec un organe complémentaire (186), en particulier avec l'actionneur, l'organe complémentaire pouvant se déplacer axialement et coopérant avec la crémaillère (188), l'organe complémentaire présentant au moins deux premiers organes d'engrènement (214, 216), dont chacun est associé à l'une desdites au moins deux rangées de dents (218, 220, 246, 248) et est pourvu d'au moins un élément d'engrènement (214', 216') par l'intermédiaire duquel il se trouve élastiquement en prise avec la rangée de dents correspondante,
un organe d'engrènement et la rangée de dents (218, 220) correspondante engrenant respectivement pleinement par l'intermédiaire dudit au moins un élément d'engrènement dudit organe d'engrènement, et au moins un autre organe d'engrènement et la rangée de dents (218,220) correspondante n'engrènant pas respectivement pleinement par l'intermédiaire dudit au moins un élément d'engrènement dudit autre organe d'engrènement.

2. Dispositif d'injection selon la revendication 1, dans lequel les rangées de dents (218, 220) de la crémaillère (188) coopérant avec l'organe complémentaire sont disposées essentiellement de façon symétrique par rapport à l'axe longitudinal de la crémaillère (188).

3. Dispositif d'injection selon la revendication 1 ou 2, dans lequel l'organe complémentaire est réalisé en tant qu'organe d'entraînement (186) pour déplacer le réservoir (136) dans le boîtier (132, 150) du dispositif d'injection.

4. Dispositif d'injection selon une ou plusieurs des revendications 1 à 3, dans lequel la crémaillère (188) présente une section transversale rectangulaire, en particulier carrée, et l'organe complémentaire présente deux organes d'engrènement (214, 216) qui coopèrent avec des rangées de dents (218, 220) situées sur des côtés opposés de cette crémaillère.

5. Dispositif d'injection selon l'une des revendications précédentes, dans lequel, en complément des premiers organes d'engrènement (214, 216) prévus sur l'organe d'actionnement déplaçable axialement, des seconds organes d'engrènement (242, 244) sont prévus qui sont également élastiquement en prise avec la crémaillère (188) par le biais d'éléments d'engrènement (242', 244'), les seconds organes d'engrènement (242, 244) étant réalisés de manière à arrêter la crémaillère (188) en position prédéfinie au cours d'un mouvement distal de l'organe d'actionnement.

6. Dispositif d'injection selon la revendication 5, dans lequel les seconds organes d'engrènement (242, 244) sont conçus de manière à permettre un mouvement de la crémaillère (188) dans le sens proximal au cours d'un mouvement proximal de l'organe d'actionnement.

7. Dispositif d'injection selon la revendication 5 ou 6, dans lequel les seconds organes d'engrènement (242, 244) sont réalisés de manière à permettre à un des seconds organes d'engrènement et à la crémaillère (188) d'engrener respectivement pleinement déjà après un déplacement prédéfini de la crémaillère (188) qui est inférieur à un pas de dent (ZT) de la crémaillère (188).

8. Dispositif d'injection selon l'une des revendications 5 à 7, dans lequel la crémaillère (188) présente une section transversale rectangulaire et de préférence carrée,
et est munie sur deux premiers côtés opposés de rangées de dents (218, 220) pour les premiers organes d'engrènement (214, 216), et sur les deux autres côtés opposés, de rangées de dents (246, 248) pour les seconds organes d'engrènement (242, 244).

9. Dispositif d'injection selon la revendication 8, dans lequel les premiers organes d'engrènement (214, 216) et les seconds organes d'engrènement (242, 244) s'engagent de manière interdigitale.

10. Dispositif d'injection selon l'une des revendications 5 à 9, dans lequel les seconds organes d'engrènement (242, 244) sont reliés à un réservoir déplaçable (136) pour le liquide injectable, ou à un support (134, 234) pour un tel réservoir.

11. Dispositif d'injection selon l'une des revendications précédentes, dans lequel les éléments d'engrènement (214', 216', 242', 244') sont respectivement conçus de manière essentiellement complémentaire aux dents des rangées de dents (218, 220, 246, 248) associées à ceux-ci.

12. Dispositif d'injection selon l'une des revendications précédentes, dans lequel un support (54, 134, 234) dont la longueur est modifiable est prévu pour la réception du réservoir (136) pour le liquide à injecter.

13. Dispositif d'injection selon la revendication 12, dans lequel le support (54, 134, 234) comprend un segment proximal (56, 134) et un segment distal (66, 234) reliés l'un à l'autre par une liaison réglable (64, 236, 240).

14. Dispositif d'injection selon la revendication 13, dans lequel la liaison réglable peut être raccourcie au moyen d'une force axiale (Fig. 8A :K) pour influencer la longueur totale du support (54, 134, 234).

15. Dispositif d'injection selon la revendication 13 ou 14, dans lequel la liaison réglable est conçue comme un dispositif à micro-crans (64, 236, 240).

16. Dispositif d'injection selon l'une des revendications 5 à 10 en lien avec une des revendications 12 à 15, dans lequel le support (134, 234) réglable au niveau de sa longueur est relié fixement aux seconds organes d'engrènement (242, 244).

17. Dispositif d'injection selon l'une des revendications 12 à 16, dans lequel le support (134, 234) peut être déplacé dans le boîtier (132) entre une position d'extrémité proximale et une position d'extrémité distale.

18. Dispositif d'injection selon l'une des revendications 1 à 11, dans lequel est prévu pour le logement du réservoir (136) contenant le liquide à injecter un support (54, 134, 234) avec un segment proximal (56, 134) et un segment distal (66, 234), ces segments étant reliés entre eux par une liaison d'enclenchement (64, 236, 240).

19. Dispositif d'injection selon la revendication 18, dans lequel la liaison d'enclenchement (64, 236, 240) comprend une pluralité de positions d'enclenchement possibles.

20. Dispositif d'injection selon la revendication 18 ou 19 en lien avec une des revendications 5 à 10, dans lequel le segment distal (234) du support (134, 234) est relié fixement aux seconds organes d'engrènement (242, 244).
